(19) Europäisches Patentamt
European Patent Office
Office européen des brevets

(11) **EP 4 099 012 B1**

(12) **EUROPEAN PATENT SPECIFICATION**

(45) Date of publication and mention
of the grant of the patent:
**04.03.2026 Bulletin 2026/10**

(21) Application number: **21747718.1**

(22) Date of filing: **27.01.2021**

(51) International Patent Classification (IPC):
*G01N 33/204* (2019.01)   *G01N 21/17* (2006.01)
*G01N 23/2251* (2018.01)   *G06T 7/00* (2017.01)
*G06V 20/69* (2022.01)   *G06V 10/764* (2022.01)
*G06V 10/54* (2022.01)   *G06V 10/36* (2022.01)
*G06V 10/50* (2022.01)   *G01N 21/84* (2006.01)
*G06N 3/09* (2023.01)

(52) Cooperative Patent Classification (CPC):
**G01N 21/84; G01N 23/2251; G01N 33/204;
G06N 3/09; G06V 10/36; G06V 10/50; G06V 10/54;
G06V 10/764; G06V 20/698;** G01N 2021/8405;
G01N 2223/418; G01N 2223/605; G06N 5/01;
G06N 7/01; G06N 20/20

(86) International application number:
**PCT/JP2021/002906**

(87) International publication number:
**WO 2021/153633 (05.08.2021 Gazette 2021/31)**

(54) **METALLOGRAPHIC STRUCTURE PHASE CLASSIFICATION METHOD, METALLOGRAPHIC STRUCTURE PHASE CLASSIFICATION DEVICE, METALLOGRAPHIC STRUCTURE PHASE LEARNING METHOD, METALLOGRAPHIC STRUCTURE PHASE LEARNING DEVICE, METALLIC MATERIAL PROPERTY PREDICTION METHOD, AND METALLIC MATERIAL PROPERTY PREDICTION DEVICE**

VERFAHREN ZUR KLASSIFIZIERUNG EINER PHASEN EINER METALLOGRAPHISCHEN STRUKTUR, VORRICHTUNG ZUR KLASSIFIZIERUNG EINER PHASEN EINER METALLOGRAPHISCHEN STRUKTUR, VERFAHREN ZUM LERNEN EINER PHASEN EINER METALLOGRAPHISCHEN STRUKTUR, VORRICHTUNG ZUM LERNEN EINER PHASEN EINER METALLOGRAPHISCHEN STRUKTUR, VERFAHREN ZUR VORHERSAGE EINER EIGENSCHAFT EINES METALLISCHEN MATERIALS, VORRICHTUNG ZUR VORHERSAGE EINER EIGENSCHAFT EINES METALLISCHEN MATERIALS

PROCÉDÉ ET DISPOSITIF DE CLASSIFICATION DE PHASE DE STRUCTURE MÉTALLOGRAPHIQUE, PROCÉDÉ ET DISPOSITIF D'APPRENTISSAGE DE PHASE DE STRUCTURE MÉTALLOGRAPHIQUE, PROCÉDÉ ET DISPOSITIF DE PRÉDICTION DE PROPRIÉTÉ DE MATÉRIAU MÉTALLIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.01.2020 JP 2020012517**

(43) Date of publication of application:
**07.12.2022 Bulletin 2022/49**

(73) Proprietor: **JFE Steel Corporation
Tokyo 100-0011 (JP)**

(72) Inventors:
• **KIYOKANE, Naoya
Tokyo 100-0011 (JP)**
• **YAMASHITA, Takako
Tokyo 100-0011 (JP)**
• **OBATA, Yoshie
Tokyo 100-0011 (JP)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**(Cont. next page)**

(74) Representative: **Hoffmann Eitle**
**Patent- und Rechtsanwälte PartmbB**
**Arabellastraße 30**
**81925 München (DE)**

(56) References cited:
| | |
|---|---|
| **WO-A1-2016/038705** | **CN-A- 103 920 717** |
| **CN-A- 110 619 355** | **DE-A1- 19 738 943** |
| **JP-A- 2012 104 042** | **JP-A- 2019 007 944** |
| **JP-A- 2019 012 037** | **JP-A- H05 248 839** |

- **GOLA JESSICA ET AL: "Advanced microstructure classification by data mining methods", COMPUTATIONAL MATERIALS SCIENCE, ELSEVIER, AMSTERDAM, NL, vol. 148, 23 March 2018 (2018-03-23), pages 324 - 335, XP085369723, ISSN: 0927-0256, DOI: 10.1016/J.COMMATSCI.2018.03.004**
- **PATRAWALA VIRAF ET AL: "Improving Histopathology Classification using Learnable Preprocessing", TENCON 2019 - 2019 IEEE REGION 10 CONFERENCE (TENCON), IEEE, 17 October 2019 (2019-10-17), pages 2460 - 2465, XP033672555, [retrieved on 20191209], DOI: 10.1109/TENCON.2019.8929391**
- **AJIOKA, FUMITO ET AL.: "Development of High Accuracy Segmentation Model for Microstructure of Steel by Deep Learning", ISIJ INTERNATIONAL ADVANCE ONLINE PUBLICATION, 18 January 2020 (2020-01-18), XP055845447, Retrieved from the Internet <URL:https://www.j-stage.jst.go.jp/article/isijinternational/advpub/0/advpub_ISIJINT-2019-568/_pdf>**
- **TOJIMA, MASANORI ET AL.: "Pattern Recognition of Microstructures using Neural Networks", TETSU-TO-HAGANE, vol. 60, no. 7, 1994, pages 59 - 64, XP000457455**
- **ADACHI, YOSHITAKA ET AL.: "Microstructure Recognition by Deep Learning", TETSU-TO-HAGANE, vol. 102, no. 12, 2016, pages 62 - 69, XP055943843**
- **BULGARE VICH, DMITRY S. ET AL.: "Automatic steel labeling on certain microstructural constituents with image processing and machine learning tools", SCIENCE AND TECHNOLOGY OF ADVANCED MATERIALS, vol. 20, no. 1, 5 June 2019 (2019-06-05), pages 532 - 542, XP055845438**
- **MULEWICZ, BARTLOMIEJ ET AL.: "Autonomous Interpretaion of the Microstructure of Steels and Special Alloys", MATERIAL SCIENCE FORUM, vol. 949, 20 March 2019 (2019-03-20), pages 24 - 31, XP055845435**

**Description**

Field

The present invention relates to a metallographic structure phase classification method, a metallographic structure phase classification device, a metallographic structure phase learning method, a metallographic structure phase learning device, a metallic material property prediction method, and a metallic material property prediction device.

Background

Recently, from the perspective of preserving the global environment, exhaust gas regulations have been implemented to reduce the amount of pollutants emitted. In addition, fuel efficiency improvement by reducing body weight has been strongly requested for automobiles. One of promising methods for reducing the weight of a vehicle body is to increase the strength of thin steel plates used for the vehicle body, and the amount of high-strength steel plates used in automobile is increasing every year.

In general, the properties of metallic materials such as steel plates strongly depend on the metallographic structure in a scale (mm to $\mu$m scale) with the optical microscope or electron microscope level, even if the metallic materials have the same composition. Thus, when developing high-strength steel plates, methods that change composition are used, such as a method that utilizes solid-solution strengthening through addition of solid-solution strengthening elements and a precipitation strengthening method that utilizes precipitates through addition of precipitation strengthening elements. In addition to these methods, other methods are used to improve mechanical properties by changing thermal treatment conditions with the same composition to change the finally realized metallographic structure.

Thus, in order to develop high-strength steel plates, it is important not only to control composition but also to control the metallographic structure. For this purpose, it is important to observe metallic materials with, for example, an optical microscope or an electron microscope and quantitatively evaluate the metallographic structure. In fact, structural observation of metallic materials under different thermal treatment conditions is ordinarily conducted in the field of material development.

After specimen preparation such as well-known polishing and etching methods is performed on metallic materials such as steel plates, the structure can be observed with a well-known image capturing device such as an electron microscope to identify each phase because the contrast is typically different for each phase. For example, when a typical steel plate consisting of ferrite and pearlite phases in iron steel materials is photographed under an optical microscope after specimen preparation by a well-known method, the ferrite phase is observed in gray contrast and the pearlite phase is observed in black contrast. Thus, the ferrite and pearlite phases can be distinguished. Through repeated observation of a metallographic structure by such a method, material development is ordinarily conducted to control the structure that changes depending on thermal treatment conditions and to try to achieve material properties requested as a material.

It is important to quantitatively evaluate the observed metallographic structure in order to link material properties to the metallographic structure. For example, it is known that the tensile strength of a typical duplex steel plate consisting of ferrite and martensite phases strongly depends on the phase fraction of the martensite phase. Conventionally, in order to quantitatively evaluate the phase fraction of these phases from images captured of the metallographic structure, each phase has been manually color-coded and the phase fraction of each phase has been measured by adding the area of each color-coded phase. However, with this method, phase recognition differs for each operator, which leads to large error among the operators, and a significantly long time is needed for manual color-coding. Thus, this method has been rarely used in practice because error is large among steel plates manufactured with the same composition and the same thermal treatment conditions and an extremely long time is needed to analyze a single image.

Luminance value binarization is an alternative quantitative evaluation method of the metallographic structure to such manual color-coding that has large error and needs a significantly long time. In this method, a luminance value threshold value is determined for an image captured of the metallographic structure and the image is converted into two colors by using a computer or the like to extract particular phases, and the area of each color is obtained to measure the phase fraction. This method can accurately classify phases when the luminance value is clearly different for each phase of the metallographic structure. In addition, since the only task is to determine the luminance value threshold value, the metallographic structure can be quantitatively evaluated much faster than with the above-described manual color-coding. On the other hand, however, metallic materials are often not accurately classified because difference in the luminance value for each phase is not clear in many cases and error is large when difference in the luminance value is not clear.

With recent significant improvement in computer performance, technologies have been developed to analyze images without including artificial elements, using more advanced calculation technologies rather than the manual color-coding or the simple luminance value binarization described above. For example, Patent Literature 1 discloses a technology as described below. First, an image of a site on a human body surface is converted into an image in an opposite color space, and each component of the image in the opposite color space is decomposed into subband images of different spatial frequencies. Then, a feature value in accordance with the site on the human body surface is calculated for the subband images, and the appearance of the site on the human body surface is evaluated based on the feature value. This technology enables objective and fast evaluation of skin condition, texture, and the like based on an image of human skin.

In addition, Patent Literature 2 discloses a technology as described below. First, a plurality of binarized images are generated by performing binarization processing a plurality of times with different binarization reference values on one metallographic image that captured the structure. Subsequently, the number of hole-shaped regions is calculated for each of the plurality of binarized images, the number of features characterizing the correspondence between a plurality of binarization reference values and the number of hole-shaped regions is specified, and output information corresponding to the number of features is generated.

Jessica Gola et al.: "Advanced microstructure classification by data mining methods", COMPUTATIONAL MATERIALS SCIENCE, ELSEVIER, AMSTERDAM, NL, vol. 148, 23 March 2018, pages 324-335, discloses the use of morphological features and the SVM to classify metallic phase of material in metallic structures.

Citation List

Patent Literature

Patent Literature 1: Japanese Patent Application Laid-open No. 2018-121752
Patent Literature 2: WO 2017/010397

Summary

Technical Problem

However, since the technology disclosed in Patent Literature 1 is a technology only for evaluating human skin, it is difficult to apply the technology to the structure of metallic materials such as steel plates. Similarly, since the technology disclosed in Patent Literature 2 is a technology only for analyzing images of living body cells, it is difficult to apply the technology to the structure of metallic materials such as steel plates.

The present invention is made in view of the above description and intended to provide a metallographic structure phase classification method, a metallographic structure phase classification device, a metallographic structure phase learning method, a metallographic structure phase learning device, a metallic material property prediction method, and a metallic material property prediction device that enable quantitative and fast evaluation of the important metallographic structure in metallic materials.

Solution to Problem

The present invention is defined by the independent claims. Preferred embodiments are defined by the dependent claims. Further aspects are provided for facilitating the understanding of the invention.

Advantageous Effects of Invention

With a metallographic structure phase learning method, a metallographic structure phase classification method, a metallographic structure phase learning device, and a metallographic structure phase classification device according to the present invention, the important metallographic structure in a metallic material can be evaluated quantitatively and speedily by determining a feature value of each phase based on an image of the metallographic structure and learning the correspondence between the feature value and the phase.

Moreover, with the metallographic structure phase learning method, the metallographic structure phase classification method, the metallographic structure phase learning device, and the metallographic structure phase classification device according to the present invention the luminance level of a metallographic image can be optimized even for different metallic materials, thereby highly accurately performing phase classification of the metallographic image.

Furthermore, with a metallic material property prediction method and a metallic material property prediction device according to the present invention, quantitative evaluation can be efficiently performed based on results of phase classification of the metallographic structure. Thus, the material properties of a metallic material can be accurately predicted by deriving the correlation between a quantitative evaluation value and the material properties of the metallic material. This allows understanding of the material properties of the metallic material simultaneously with visual recognition of an image of a metallographic structure, thereby improving the efficiency of metallic material (for example, steel plate) development.

Brief Description of Drawings

FIG. 1 is a block diagram illustrating an exemplary schematic configuration of a metallographic structure phase learning device according to an embodiment of the present invention.

FIG. 2 is a block diagram illustrating an exemplary schematic configuration of a metallographic structure phase classification device according to a first embodiment of the present invention.

FIG. 3 is a block diagram illustrating an exemplary schematic configuration of a metallographic structure phase classification device according to a second embodiment of the present invention.

FIG. 4 is a flowchart illustrating the process of a metallographic structure phase learning method according to the embodiment of the present invention.

FIG. 5 is a flowchart illustrating the process of a metallographic structure phase classification method according to the first embodiment of the present invention.

FIG. 6 is a flowchart illustrating the process of a metallographic structure phase classification method according to the second embodiment of the present invention.

FIG. 7 is a block diagram illustrating an exemplary schematic configuration of a metallic material property prediction device according to the embodiment of the present invention.

FIG. 8 is a flowchart illustrating the process of a metallic material property prediction method according to the embodiment of the present invention.

FIG. 9 is an example of the metallographic structure phase learning method according to the present invention and a diagram illustrating an image captured by a scanning electron microscope in an image inputting process and a line profile of the luminance value of the image.

FIG. 10 is an example of the metallographic structure phase learning method according to the present invention and a diagram illustrating an image after background removal is performed in an image preprocessing process and a line profile of the luminance value of the image.

FIG. 11 is an example of the metallographic structure phase learning method according to the present invention and a diagram illustrating ferrite and martensite phases designated in a phase designation process.

FIG. 12 is an example of the metallographic structure phase learning method according to the present invention and a diagram illustrating an identity feature value, a Gaussian feature value, and a Mean feature value calculated in a feature value calculation process.

FIG. 13 is an example of the metallographic structure phase learning method according to the present invention and a diagram illustrating a decision tree generated in a model generation process.

FIG. 14 is an example of the metallographic structure phase classification method according to the present invention and a diagram illustrating an identity feature value, a Gaussian feature value, and a Mean feature value calculated in the feature value calculation process.

FIG. 15 is an example of the metallographic structure phase classification method according to the present invention and a diagram illustrating an original image, a classification result of the example, and a classification result of a comparative example.

FIG. 16 is an example of the metallographic structure phase classification method according to the present invention and a diagram illustrating an original image, an image after background removal, a classification result of the example, and a classification result of the comparative example.

FIG. 17 is an example of a metallic material property prediction method according to the present invention and a diagram illustrating a histogram of ferrite phase circularity calculated from metallographic images of (b) of FIG. 15 and (c) of FIG. 16.

FIG. 18 is an example of the metallic material property prediction method according to the present invention and a diagram illustrating a result of tensile strength prediction using a prediction model (neural network model) generated by a prediction model generation unit.

Description of Embodiments

The following describes a metallographic structure phase classification method, a metallographic structure phase classification device, a metallographic structure phase learning method, a metallographic structure phase learning device, a metallic material property prediction method, and a metallic material property prediction device according to embodiments of the present invention with reference to the accompanying drawings.

A metallographic structure phase learning method and a metallographic structure phase learning device according to the present embodiment are a method and a device that learn the phase of the metallographic structure, which is important information for controlling the properties of a metallic material used as various product materials such as structural members and automobile members. In addition, a metallographic structure phase classification method and a metallographic structure phase classification device according to the present embodiment are a method and a device that classify the phase of the metallographic structure based on results of learning by the metallographic structure phase learning method and learning device. Note that, a metallic material for which phase learning and classification are performed in the present embodiment is, for example, a duplex steel plate (DP steel plate) made of ferrite and martensite phases. In the following, the configurations of a metallographic structure phase learning device and a metallographic structure phase classification device will be described, and then learning method and classification methods using these devices will be described. Then, the configuration of a metallic material property prediction device will be described, and then a material property prediction method using this device will be described.

(Metallographic structure phase learning device)

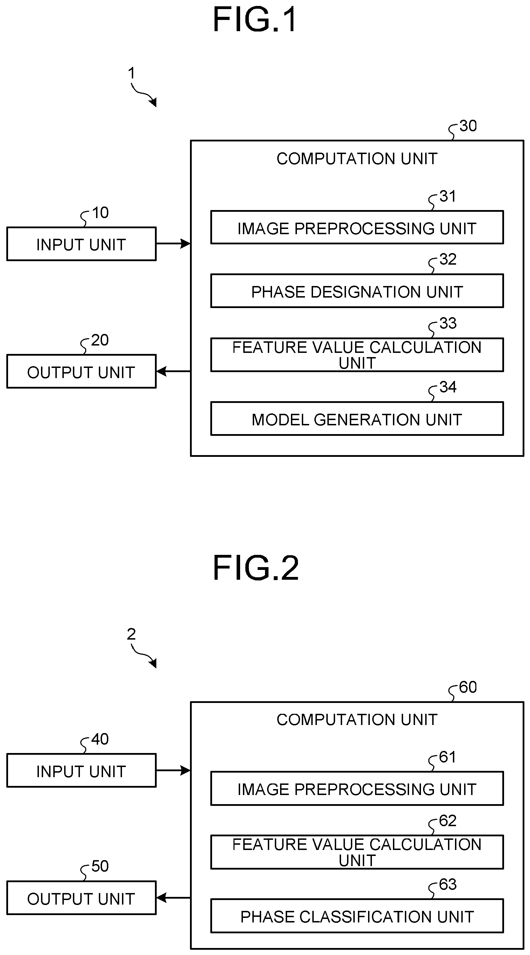

A metallographic structure phase learning device (hereinafter referred to as a "learning device") 1 will be described with reference to FIG. 1. The learning device 1 includes an input unit 10, an output unit 20, and a computation unit 30.

The input unit 10 is an input unit that inputs, to the computation unit 30, an image (hereinafter referred to as a "metallographic image") that captured the metallographic structure of a metallic material. This metallographic image may be acquired by a well-known image capturing device, such as an optical microscope or a scanning electron microscope, which is widely used for image capturing of the metallographic image.

Typically, specimen preparation is performed for image capturing of a metallographic image, and a well-known method may be used for this specimen preparation. For example, in a case of image capturing of a scanning electron microscope, a well-known specimen preparation is exemplified with a method as follows. First, a specimen piece is roughly polished with emery paper, and then the surface of the specimen is polished to a mirror by buffing using alumina abrasive of $0.5\ \mu m$ to $2.0\ \mu m$. Then, the specimen piece is eroded in a dilute corrosive solution such as 1% Nital for approximately one second. This specimen preparation is not essential, but mirror polishing is preferably performed until any existing flaw on the specimen surface disappears.

Furthermore, image capturing conditions such as the magnification at image capturing of a metallographic image, the intensity of a light source of an optical microscope, and the acceleration voltage of an electron microscope are not particularly limited, and may be set by an operator in accordance with a metallographic structure as an analysis target. In addition, at image capturing of a metallographic image, contrast adjustment is preferably performed before the image capturing so that the contrast for each phase is clear. Note that, a metallographic image is not limited to acquisition from an image capturing device, and may be acquired from an external or internal recording medium (such as a hard disk or a USB memory) in which a captured image is stored in advance and may be input to the input unit 10.

The output unit 20 is an output unit that outputs results of computation by the computation unit 30. The output unit 20 is configured as, for example, a display, a printer, or a smartphone. The output unit 20 outputs, for example, a metallographic image preprocessed by an image preprocessing unit 31, a feature value of the phase of the metallographic structure, which is calculated by a feature value calculation unit 33, and a result of learning of each phase of the metallographic structure by a model generation unit 34. Note that, the outputting by the output unit 20 is not limited to a particular format, and may be performed in, for example, the format of data such as a text file or an image file or the format of projection to an output device.

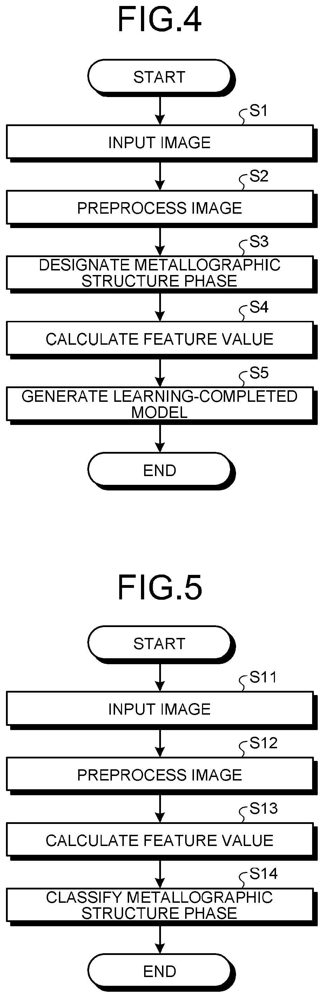

The computation unit 30 is implemented with a processor made of a central processing unit (CPU), and a memory (main storage unit) made of a random access memory (RAM), a read only memory (ROM), or the like. The computation unit 30 loads a computer program onto a work area of the main storage unit, executes the computer program, and controls each component or the like through execution of the computer program, thereby achieving a function that matches a predetermined purpose. In addition, through the above-described computer program execution, the computation unit 30 functions as the image preprocessing unit 31, a phase designation unit 32, the feature value calculation unit 33, and the model generation unit 34. Note that, each component will be described later in detail (refer to FIG. 4).

(Metallographic structure phase classification device (first embodiment))

A metallographic structure phase classification device (hereinafter referred to as a "classification device") 2 according to a first embodiment will be described with reference to FIG. 2. The classification device 2 includes an input unit 40, an output unit 50, and a computation unit 60.

The input unit 40 is an input unit that inputs a metallographic image of a metallic material to the computation unit 60. This metallographic image may be acquired by a well-known image capturing device, such as an optical microscope or a scanning electron microscope, which is widely used for image capturing of the metallographic image. Similarly to a metallographic image input to the input unit 10, a metallographic image input to the input unit 40 is subjected to specimen preparation. The method of the specimen preparation is same as the method of the specimen preparation for a metallographic image input to the input unit 10. In addition, image capturing conditions at image capturing of a metallographic image input to the input unit 40, contrast adjustment, and metallographic image acquisition from a recording medium are same as in the case of a metallographic image input to the input unit 10.

The output unit 50 is an output unit that outputs results of computation by the computation unit 60. The output unit 50 is configured as, for example, a display, a printer, or a smartphone. The output unit 50 outputs, for example, a metallographic image preprocessed by an image preprocessing unit 61, a feature value of each phase of the metallographic structure, which is calculated by a feature value calculation unit 62, and results of phase classification of the metallographic structure by a phase classification unit 63. Note that, the outputting by the output unit 50 is not limited to a particular format, and may be performed in, for example, the format of data such as a text file or an image file or the format of projection to an output device.

Similarly to the computation unit 30, the computation unit 60 is implemented with a processor made of a CPU or the like, and a memory made of a RAM, a ROM, or the like. The computation unit 60 loads a computer program onto a work area of the main storage unit, executes the computer program, and controls each component or the like through execution of the computer program, thereby achieving a function that matches a predetermined purpose. In addition, through the above-described computer program execution, the computation unit 60 functions as the image preprocessing unit 61, the feature value calculation unit 62, and the phase classification unit 63. Note that, each component will be described later in detail (refer to FIG. 5).

(Metallographic structure phase classification device (second embodiment))

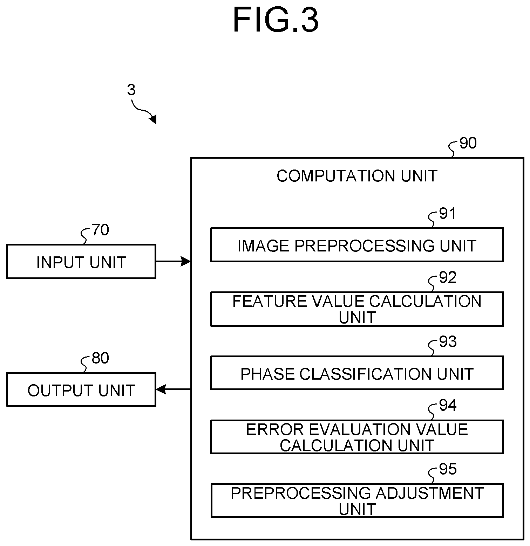

A metallographic structure phase classification device (hereinafter referred to as a "classification device") 3 according to a second embodiment will be described with reference to FIG. 3. The classification device 3 includes an input unit 70, an output unit 80, and a computation unit 90.

The input unit 70 is an input unit that inputs a metallographic image of a metallic material to the computation unit 90. This metallographic image may be acquired by a well-known image capturing device, such as an optical microscope or a scanning electron microscope, which is widely used for image capturing of the metallographic image. Similarly to metallographic images input to the input units 10 and 40, a metallographic image input to the input unit 70 is subjected to specimen preparation. The method of the specimen preparation is same as the method of the specimen preparation for metallographic images input to the input units 10 and 40. In addition, image capturing conditions at image capturing of a metallographic image input to the input unit 70, contrast adjustment, and metallographic image acquisition from a recording medium are same as in the case of metallographic images input to the input units 10 and 40.

The output unit 80 is an output unit that outputs results of computation by the computation unit 90. The output unit 80 is configured as, for example, a display, a printer, or a smartphone. The output unit 80 outputs, for example, a metallographic image preprocessed by an image preprocessing unit 91, a feature value of each phase of the metallographic structure, which is calculated by a feature value calculation unit 92, and results of phase classification of the metallographic structure by a phase classification unit 93. In addition, the output unit 80 outputs, for example, an error evaluation value calculated by an error evaluation value calculation unit 94, and preprocessing conditions (preprocessing parameters) adjusted by a preprocessing adjustment unit 95. Note that, the outputting by the output unit 80 is not limited to a particular format, and may be performed in, for example, the format of data such as a text file or an image file or the format of projection to an output device.

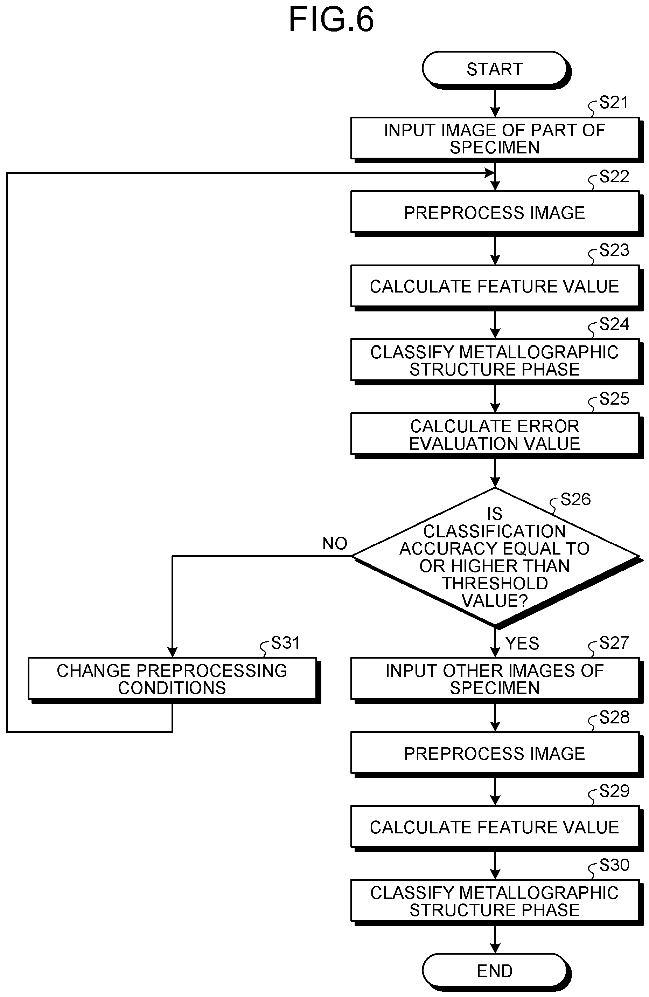

Similarly to the computation units 30 and 60, the computation unit 90 is implemented with a processor made of a CPU or the like, and a memory made of a RAM, a ROM, or the like. The computation unit 90 loads a computer program onto a work area of the main storage unit, executes the computer program, and controls each component or the like through execution of the computer program, thereby achieving a function that matches a predetermined purpose. In addition, the computation unit 90 functions as the image preprocessing unit 91, the feature value calculation unit 92, the phase classification unit 93, the error evaluation value calculation unit 94, and the preprocessing adjustment unit 95 through the above-described computer program execution. Note that, each component will be described later in detail (refer to FIG. 6).

(Metallographic structure phase learning method)

The metallographic structure phase learning method using the learning device 1 will be described with reference to FIG. 4. The metallographic structure phase learning method performs an image inputting process, an image preprocessing process, a phase designation process, a feature value calculation process, and a model generation process in the stated order. Note that, a specimen preparation process that the surface of a specimen is, for example, polished by a well-known method, and an image capturing process in which a metallographic image of a metallic material is captured by a well-known image capturing device such as an optical microscope or a scanning electron microscope may be performed before the image preprocessing process as necessary.

<Image inputting process>

In the image inputting process, the input unit 10 inputs a metallographic image provided with specimen preparation as necessary to the computation unit 30 (step S1).

<Image preprocessing process>

In the image preprocessing process, the image preprocessing unit 31 preprocesses the metallographic image (step S2). The contrast of the metallographic image (original image) input by the input unit 10 is often different among captured images due to difference in a specimen preparation method before image capturing, and the strength of a light source and contrast adjustment at image capturing, even in a case of image capturing of the same specimen by the same image capturing device. In addition, the luminance value of the metallographic image input by the input unit 10 is different among positions in some cases even in the same phase in the same image due to difference in the position of a light source in a case of an optical microscope, difference in the position of a detector in a case of an electron microscope, and the like. Such difference in the luminance value reduces learning efficiency and also causes error in phase classification. Thus, in the image preprocessing process, the metallographic image is preprocessed by adjusting the luminance value of each pixel of the metallographic image.

Examples of the metallographic image preprocessing include pieces of processing (1) to (3) below. A plurality of the pieces of processing (1) to (3) below may be combined, or only one (for example, identity processing) thereof may be performed as preprocessing. However, in a case of image capturing of a metallographic structure, the luminance value is different for each metallographic image or the luminance value is different among positions in the same image in many cases, and thus at least background removal processing among the pieces of processing (1) to (3) below is preferably performed. Processing that removes maximum and minimum values of the entire luminance value by a predetermined range may be performed in addition to the pieces of processing (1) to (3) below.

(1) Identity processing

In the identity processing, an input metallographic image is directly returned as an output.

(2) Background removal processing

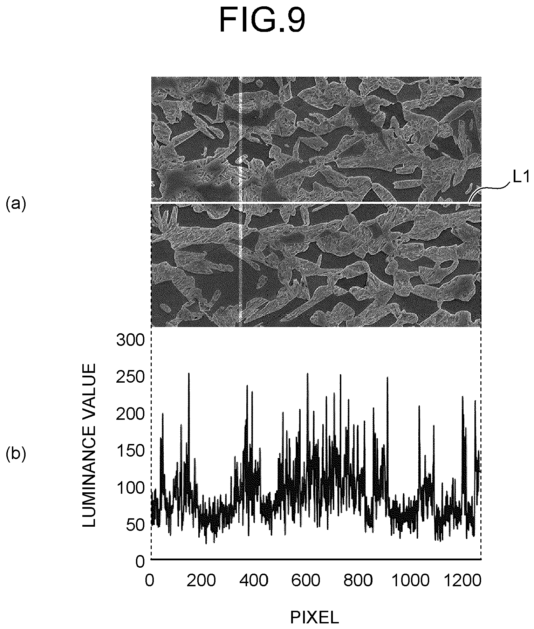

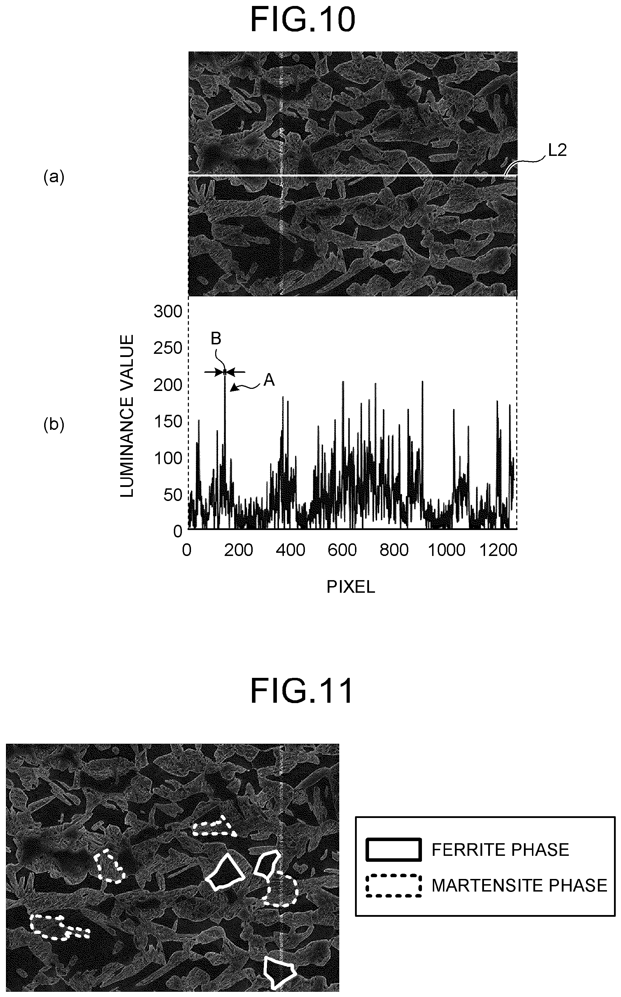

In the background removal processing, the mean luminance value (background) of a metallographic image is removed. The entire metallographic image captured by an optical microscope or an electron microscope has a certain luminance value or higher and the absolute value of the luminance value of each metallographic image is largely different in some cases. Furthermore, some parts appear bright and some other parts appear dark due to difference in the position of a light source or a detector in some cases. Thus, in the background removal processing, the luminance value is adjusted by determining a rectangular region of x pixels $\times$ y pixels in advance, calculating the minimum value of the luminance value within the rectangular region, performing interpolation between pixels with an appropriate function (for example, a linear function), and subtracting the interpolation function from the luminance values of all pixels. Note that, "the pixel number x" and "the number of pixels y" may be optionally set for each metallographic image by the operator but are preferably set to, for example, a size approximately equal to a smaller crystal grain size among a plurality of phases of the metallographic structure. Furthermore, when there is anisotropy, "the pixel number x" and "the pixel number y" are preferably set to sizes approximately equal to crystal grain sizes in the x and y directions. Furthermore, the region of x pixels $\times$ y pixels does not need to be rectangular, and for example, the region of x pixels $\times$ y pixels preferably has a spherical shape when a metallographic image has a spherical shape. Note that, the background removal processing will be described later in detail (refer to FIGS. 9 and 10).

(3) Histogram equalization processing

In histogram equalization processing, a histogram of the luminance value of a metallographic image is equalized. Distribution of the luminance value of a metallographic image captured by an optical microscope or an electron microscope has a largely different mean value and standard deviation for each metallographic image in many cases. Thus, standardization that aligns the mean value and standard deviation of the histogram is performed to homogenize the mean value and variance of the luminance value of the metallographic image.

<Phase designation process>

In the phase designation process, the phase designation unit 32 applies the label of one or a particular metallographic structure phase designated in advance to any pixel corresponding to the phase in a metallographic image captured of the metallographic structure of a metallic material (step S3). Note that, the "application of the label of each phase to any pixel of the phase in a metallographic image" is to specify any pixel corresponding to the ferrite or martensite phase in the metallographic image and associate the pixel in the metallographic image with the ferrite or martensite phase in a case of, for example, a DP steel plate. Note that, the phase designation process will be described later in detail (refer to FIG. 11).

<Feature value calculation process>

In the feature value calculation process, the feature value calculation unit 33 calculates one or more feature values for each pixel to which the label of a phase is allocated in the phase designation process (step S4). In the feature value calculation process, for example, one or more feature values among feature values (1) to (8) below are calculated.

(1) Identity feature value

The identity feature value is a feature value representing the luminance value of a metallographic image.

(2) Mean feature value

The Mean feature value is a feature value representing the mean value of the luminance value in a predetermined range of a metallographic image. Specifically, the Mean feature value is obtained by taking a predetermined range of "the pixel number $x \times$ the pixel number $y$" out of each phase in a metallographic image and averaging the luminance value in the range. "The pixel number $x$" and "the pixel number $y$" may be equal sizes or different sizes. Moreover, the range of "the pixel number $x$" and "the pixel number $y$" is preferably, for example, a range that is larger than noise included in a metallographic image and includes a phase of a size smaller than 1/2 of a smaller crystal grain size among a plurality of phases of the metallographic structure. Furthermore, when there is anisotropy, "the pixel number $x$" and "the pixel number $y$" are preferably set to sizes approximately equal to crystal grain sizes in the x and y directions. Furthermore, the region of x pixels $\times$ y pixels does not need to be rectangular, and for example, the region of x pixels $\times$ y pixels preferably has a spherical shape when a metallographic image has a spherical shape. Moreover, the Mean feature value may be calculated for a plurality of pixel numbers x and y. Note that, when the pixel range is too large, the Mean feature value is affected by grain boundaries and other adjacent phases. Thus, the pixel range is preferably a range that includes a size smaller than 1/2 of a larger crystal grain size.

"Noise" included in a metallographic image indicates, for example, a part at which the luminance value is abruptly high in a metallographic image (refer to part A in (b) of FIG. 10, for example). "The pixel numbers x and y larger than noise" indicates being larger than a noise width (refer to part B in (b) of FIG. 10).

(3) Gaussian feature value

The Gaussian feature value is a feature value representing the mean value of the luminance value having a larger weight at a position closer to the center in a predetermined range of a metallographic image. Specifically, the Gaussian feature value is obtained by taking a predetermined range of "the pixel number $x \times$ the number $y$ of pixels" out of each phase in a metallographic image and taking out the mean value of a pixel having a larger weight at a position closer to the center. "The pixel number $x$" and "the pixel number $y$" may be equal sizes or different sizes. Moreover, the range of "the pixel number $x$" and "the pixel number $y$" is preferably, for example, a range that is larger than noise included in a metallographic image and includes a phase of a size smaller than 1/2 of a smaller crystal grain size among a plurality of phases of the metallographic structure. Furthermore, when there is anisotropy, "the pixel number $x$" and "the pixel number $y$" are preferably set to sizes approximately equal to crystal grain sizes in the x and y directions. Furthermore, the region of x pixels $\times$ y pixels does not need to be rectangular, and for example, the region of x pixels $\times$ y pixels preferably has a spherical shape when a metallographic image has a spherical shape. Moreover, the Gaussian feature value may be calculated for a plurality of pixel numbers x and y. Note that, when the pixel range is too large, the Gaussian feature value is affected by grain boundaries and other adjacent phases. Thus, the pixel range is preferably a range that includes a size smaller than 1/2 of a larger crystal grain size.

Weights applied to central pixels when the Gaussian feature value is calculated may be optionally set by the operator, but a Gauss function expressed in Expression (1) below is preferably used.

$$A \cdot \exp\left(-(\Delta x^2 + \Delta y^2)\right) \tag{1}$$

Note that, $\Delta x$ and $\Delta y$ in Expression (1) above can be expressed in Expressions (2) and (3) below.

$$\Delta x = x_i - x_c \tag{2}$$

$$\Delta y = y_i - y_c \tag{3}$$

where $x_c$, $y_c$ are central coordinates of a rectangle, and
$x_i, y_i$ are positional coordinates of a rectangle.

(4) Median feature value

A Median feature value is a feature value representing the center value of the luminance value in a predetermined range of a metallographic image. Specifically, the Median feature value is obtained by taking a predetermined range of "the pixel number x × the number y of pixels" out of each phase in a metallographic image and taking out the median of the luminance value in the range. "The pixel number x" and "the pixel number y" may be equal sizes or different sizes. Moreover, the range of "the pixel number x" and "the pixel number y" is preferably, for example, a range that is larger than noise included in a metallographic image and includes a phase of a size smaller than 1/2 of a smaller crystal grain size among a plurality of phases of the metallographic structure. Furthermore, when there is anisotropy, "the pixel number x" and "the pixel number y" are preferably set to sizes approximately equal to crystal grain sizes in the x and y directions. Furthermore, the region of x pixels × y pixels does not need to be rectangular, and for example, the region of x pixels × y pixels preferably has a spherical shape when a metallographic image has a spherical shape. Furthermore, the Median feature value may be calculated for a plurality of pixel numbers x and y. Note that, when the pixel range is too large, the Median feature value is affected by grain boundaries and other adjacent phases. Thus, the pixel range is preferably a range that includes a size smaller than 1/2 of a larger crystal grain size.

(5) Max feature value

A Max feature value is a feature value representing the maximum value of the luminance value in a predetermined range of a metallographic image. Specifically, the Max feature value is obtained by taking a predetermined range of "the pixel number x × the number y of pixels" out of each phase in a metallographic image and taking out the maximum value of the luminance value in the range. "The pixel number x" and "the pixel number y" may be equal sizes or different sizes. Moreover, the range of "the pixel number x" and "the pixel number y" is preferably, for example, a range that is larger than noise included in a metallographic image and includes a phase of a size smaller than 1/2 of a smaller crystal grain size among a plurality of phases of the metallographic structure. Furthermore, when there is anisotropy, "the pixel number x" and "the pixel number y" are preferably set to sizes approximately equal to crystal grain sizes in the x and y directions. Furthermore, the region of x pixels × y pixels does not need to be rectangular, and for example, the region of x pixels × y pixels preferably has a spherical shape when a metallographic image has a spherical shape. Moreover, the Max feature value may be calculated for a plurality of pixel numbers x and y. Note that, when the pixel range is too large, the Max feature value is affected by grain boundaries and other adjacent phases. Thus, the pixel range is preferably a range that includes a size smaller than 1/2 of a larger crystal grain size.

(6) Min feature value

A Min feature value is a feature value representing the minimum value of the luminance value in a predetermined range of a metallographic image. Specifically, the Min feature value is obtained by taking a predetermined range of "the pixel number x × the number y of pixels" out of each phase in a metallographic image and taking out the minimum value of the luminance value in the range. "The pixel number x" and "the pixel number y" may be equal sizes or different sizes. Moreover, the range of "the pixel number x" and "the pixel number y" is preferably, for example, a range that is larger than noise included in a metallographic image and includes a phase of a size smaller than 1/2 of a smaller crystal grain size among a plurality of phases of the metallographic structure. Furthermore, when there is anisotropy, "the pixel number x" and "the pixel number y" are preferably set to sizes approximately equal to crystal grain sizes in the x and y directions. Furthermore, the region of x pixels × y pixels does not need to be rectangular, and for example, the region of x pixels × y pixels preferably has a spherical shape when a metallographic image has a spherical shape. Moreover, the Min feature value may be calculated for a plurality of pixel numbers x and y. Note that, when the pixel range is too large, the Min feature value is affected by grain boundaries and other adjacent phases. Thus, the pixel range is preferably a range that includes a size smaller than 1/2 of a larger crystal grain size.

(7) Derivative feature value

A Derivative feature value is obtained by taking a predetermined range of "the pixel number x × the number y of pixels" out of each phase in a metallographic image and calculating differential values for end pixels in the range in the x and y directions. "The pixel number x" and "the pixel number y" may be equal sizes or different sizes. Moreover, the range of "the pixel number x" and "the pixel number y" is preferably, for example, a range that is larger than noise included in a metallographic image and includes a phase of a size smaller than 1/2 of a smaller crystal grain size among a plurality of phases of the metallographic structure. Furthermore, when there is anisotropy, "the pixel number x" and "the pixel number y" are preferably set to sizes approximately equal to crystal grain sizes in the x and y directions. Furthermore, the region of x pixels × y pixels does not need to be rectangular, and for example, the region of x pixels × y pixels preferably has a spherical shape when a metallographic image has a spherical shape. Moreover, the Derivative feature value may be calculated for a plurality of pixel numbers x and y. Note that, when the pixel range is too large, the Derivative feature value is affected by grain boundaries and other adjacent phases. Thus, the pixel range is preferably a range that includes a size smaller than 1/2 of a larger crystal grain size.

(8) Derivative addition feature value

A Derivative addition feature value is obtained by convolving the Derivative feature value through computation of the Mean feature value, the Gaussian feature value, the Median feature value, the Max feature value, and the Min feature value described above for the Derivative feature value described above. "The pixel number x" and "the pixel number y" described above may be equal sizes or different sizes. Moreover, the range of "the pixel number x" and "the pixel number y" is preferably, for example, a range that is larger than noise included in a metallographic image and includes a phase of a size smaller than 1/2 of a smaller crystal grain size among a plurality of phases of the metallographic structure. Furthermore, when there is anisotropy, "the pixel number x" and "the pixel number y" are preferably set to sizes approximately equal to crystal grain sizes in the x and y directions. Furthermore, the region of x pixels × y pixels does not need to be rectangular, and for example, the region of x pixels × y pixels preferably has a spherical shape when a metallographic image has a spherical shape. Moreover, the Derivative addition feature value may be calculated for a plurality of pixel numbers x and y. Note that, when the pixel range is too large, the Derivative addition feature value is affected by grain boundaries and other adjacent phases. Thus, the pixel range is preferably a range that includes a size smaller than 1/2 of a larger crystal grain size.

Since the above-described feature values (1) to (6) are computed for a large number of pixels of each phase, the same phase has different feature values and histograms of the feature values can be produced for each phase. The above-described feature values (1) to (6) may be all calculated or only some feature values may be calculated. Furthermore, a feature value obtained through a combination of the feature value computations may be added, and a feature value not described above may be added as necessary. Selection thereof is preferably made by the operator so that learning accuracy in a learning method improves, and a feature value that largely differs among phases is preferably employed. For example, in histograms (frequency distributions) obtained for feature values of phases, the difference (referred to as a distance D) between values of the feature value at which the frequency of the feature value is highest for the phases is normalized by the difference between the maximum and minimum values of the feature value. Then, it is thought that the normalized distance D is compared among feature values, and, for example, that a plurality of feature values having the larger normalized distance D are employed.

Furthermore, the above-described feature values (7) and (8) may be calculated in addition to the feature values (1) to (6) or a feature value as a combination of the feature values may be added. Since, similarly to the above-described feature values (1) to (6), the feature values (7) and (8) are computed for a large number of pixels of each phase, the same phase has different feature values and histograms of the feature values can be produced for each phase.

Note that, when the above-described feature values (1) to (8) are each calculated, the predetermined range of "the pixel number x × the number y of pixels" is taken out and the feature value is calculated to obtain a feature value convolved for a pixel at the center of "the pixel number x × the number y of pixels". Then, the feature value is calculated at each position while "the pixel number x × the number y of pixels" is moved in a metallographic image. Furthermore, when "the pixel number x × the number y of pixels" is positioned at an end (upper, lower, right, or left end) of the metallographic image, the feature value is calculated by applying a boundary condition or limiting the pixel numbers to the range of the center to the end. As for the boundary condition, the same feature value at the center of "the pixel number x × the number y of pixels" is applied to pixels outside the center of "the pixel number x × the number y of pixels". Alternatively, the feature value is calculated by applying extrapolation from the center toward the outside by using an extrapolation function such as a linear function, an exponential function, or a spline function.

<Model generation process>

In the model generation process, the model generation unit 34 generates a learning-completed model by performing learning (machine learning) using a feature value calculated in the feature value calculation process for a pixel to which the label of each phase is allocated as an input and the label of each phase as an output (step S5). In the model generation process, a decision tree in which a feature value is set as a branching condition is generated. Note that, although the method of the machine learning in the model generation process is not limited to a decision tree, and may be, for example, a random forest or a neural network, description in the present embodiment is made on a decision tree as an example.

Specifically, in the model generation process, binarization is repeated a plurality of times to classify the phase of the metallographic structure based on the feature value calculated for each phase in the feature value calculation process. In this case, the accuracy at which classification of each phase is to be performed is set in advance based on a phase designated by the operator and the feature value calculated for each phase in the feature value calculation process, and then branch learning through binarization is performed based on this set numerical value information.

For example, when binarization branching is set to be performed at the accuracy of 80%, a decision tree is produced by performing learning through a plurality of repetitions of feature value binarization so that phase classification is performed based on a designated phase and its feature value at the probability of 80% or higher. The above-described accuracy setting may be optionally performed by the operator, but its lower limit is preferably set to be 80% or higher. The classification accuracy decreases when the accuracy is set to be lower than 80%. However, when the accuracy is set to be too high, the classification accuracy degrades due to overlearning in image classification after learning. Thus, the upper limit of the accuracy is preferably lower than 99%.

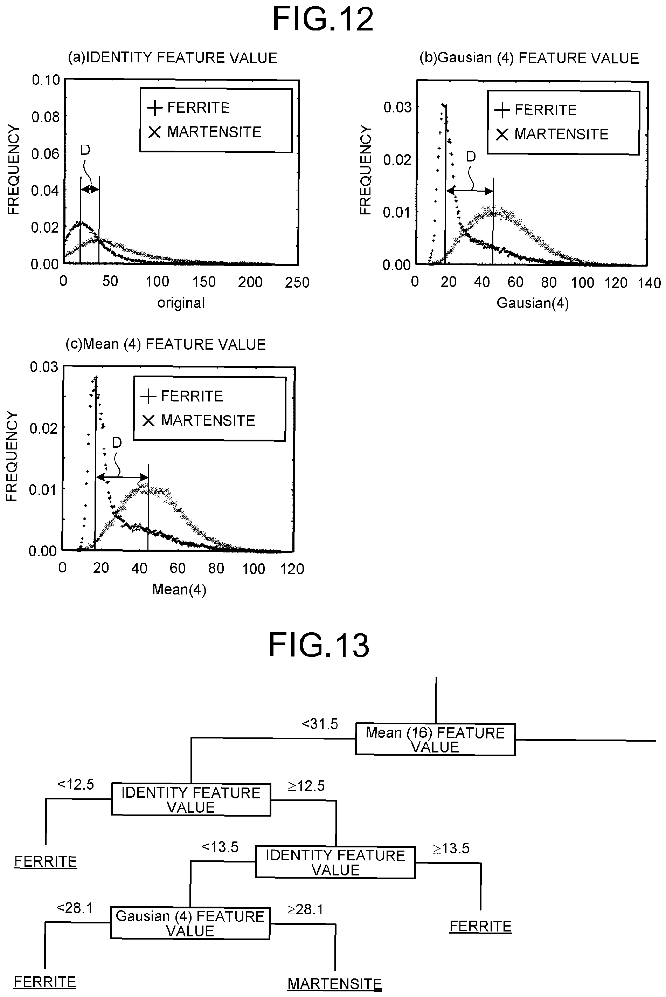

When binarization is performed a plurality of times in the model generation process, the binarization order (branching order) of each feature value may be designated by the operator in advance or may be determined at random by using a random number. Since the optimum binarization order of each feature value is often unknown in advance, the binarization order of each feature value for classification at an accuracy equal to or higher than the above-described accuracy is preferably searched by a computer by using a random number. Similarly, since the optimum number of times of binarization of each feature value is often unknown in advance, the number of times of binarization of each feature value for classification at an accuracy equal to or higher than the above-described accuracy is preferably searched by a computer. Furthermore, a feature value used as a branching condition at binarization may be used as a branching condition a plurality of times. Note that, the model generation process will be described later in detail (refer to FIG. 13). Moreover, a feature value as model input may be selected based on the distance D (including the normalized distance) between the maximum values of the frequency of the above-described feature value in each phase, and more specifically, the selection may be performed in descending order of the distance D to obtain highest model accuracy.

After the feature value calculation process is performed at step S4, each feature value calculation result may be output from the output unit 20. The outputting is not limited to a particular format, and may be performed in the format of a text file or an image file. When each feature value calculation result is output in the format of a text file or an image file in this manner, the degree of classification with each feature value can be checked before phase learning is performed. Similarly, after a learning-completed model generation process is performed at step S5, a learning-completed model generation result (for example, a decision tree) may be output from the output unit 20. The outputting is not limited to a particular format, and may be performed in the format of a text file or an image file.

With the metallographic structure phase learning device and the metallographic structure phase learning method according to the embodiment as described above, the important metallographic structure in a metallic material can be evaluated quantitatively and speedily by calculating a feature value of each phase from an image of the metallographic structure and learning the correspondence between the feature value and the phase.

Moreover, with the metallographic structure phase learning device and the metallographic structure phase learning method according to the embodiment, image preprocessing is appropriately performed on a captured metallographic image, the phase of a metallographic image on which the image preprocessing is performed is designated, and a feature value of the designated phase is calculated and recorded. Then, appropriate binarization is repeatedly performed on the recorded feature value of each phase to learn a repeated binarization procedure. Then, phase classification of an optional metallographic image is automatically performed based on results of the phase learning, thereby accurately measuring the phase fraction of each phase.

(Metallographic structure phase classification method (first embodiment))

A metallographic structure phase classification method using the classification device 2 will be described with reference to FIG. 5. The metallographic structure phase classification method performs an image inputting process, an image preprocessing process, a feature value calculation process, and a phase classification process in the stated order. Note that, a specimen preparation process that the surface of a specimen is, for example, polished by a well-known method, and an image capturing process in which a metallographic image of a metallic material is captured by a well-known image capturing device such as an optical microscope or a scanning electron microscope may be performed before the image preprocessing process as necessary.

<Image inputting process>

In the image inputting process, the input unit 40 inputs a metallographic image provided with specimen preparation as necessary to the computation unit 60 (step S11).

<Image preprocessing process>

In the image preprocessing process, the image preprocessing unit 61 preprocesses the metallographic image (step S12). The contrast of the metallographic image (original image) input by the input unit 40 is often different among captured images due to difference in a specimen preparation method before image capturing, and the strength of a light source and contrast adjustment at image capturing, even in a case of image capturing of the same specimen by the same image capturing device. In addition, the luminance value of the metallographic image input by the input unit 40 is different among positions in some cases even in the same phase in the same image due to difference in the position of a light source in a case of an optical microscope, difference in the position of a detector in a case of an electron microscope, and the like. Such difference in the luminance value reduces learning efficiency and also causes error in phase classification. Thus, in the image preprocessing process, the metallographic image is preprocessed by adjusting the luminance value of each pixel of the metallographic image.

Similarly to the preprocessing process (step S2 in FIG. 4) of the above-described learning method, examples of metallographic image preprocessing include (1) identity processing, (2) background removal processing, and (3) histogram equalization processing. Note that, the contents of these pieces of processing are same as those of the preprocessing process of the above-described learning method, and thus description thereof is omitted. A plurality of the above-described pieces of processing (1) to (3) may be combined, or only one (for example, the identity processing) thereof may be performed as preprocessing. However, in a case of image capturing of a metallographic structure, the luminance value is different for each metallographic image or the luminance value is different among positions in the same image in many cases, and thus at least the background removal processing among the above-described pieces of processing (1) to (3) is preferably performed.

<Feature value calculation process>

In the feature value calculation process, the feature value calculation unit 62 calculates one or more feature values for each pixel of a metallographic image input in the image inputting process (step S13). In the feature value calculation process, similarly to the feature value calculation process (step S4 in FIG. 4) of the above-described learning method, one or more feature values among (1) the identity feature value, (2) the Mean feature value, (3) the Gaussian feature value, (4) the Median feature value, (5) the Max feature value, and (6) the Min feature value are calculated.

Furthermore, in the feature value calculation process, similarly to the feature value calculation process (step S4 in FIG. 4) of the above-described learning method, one or more feature values among the feature values (1) to (8), additionally including (7) the Derivative feature value and (8) the Derivative addition feature value may be calculated. Moreover, in the feature value calculation process, the feature values (7) and (8) may be calculated in addition to the feature values (1) to (6).

Since the above-described feature values (1) to (6) are computed for a large number of pixels of each phase, the same phase has different feature values and histograms of the feature values can be produced for each phase. The above-described feature values (1) to (6) may be all calculated or only some feature values may be calculated. Furthermore, a feature value obtained through a combination of the feature value computations may be added, and a feature value not described above may be added as necessary. Selection thereof is preferably made by the operator so that learning accuracy in a learning method improves, and a feature value that largely differs among phases is preferably employed.

Furthermore, since, similarly to the above-described feature values (1) to (6), the feature values (7) and (8) are computed for a large number of pixels of each phase, the same phase has different feature values and histograms of the feature values can be produced for each phase. The above-described feature values (1) to (8) may be all calculated or only some feature values may be calculated. Furthermore, a feature value obtained through a combination of the feature value computations may be added, and a feature value not described above may be added as necessary. Selection thereof is preferably made by the operator so that learning accuracy in a learning method improves, and a feature value that largely differs among phases is preferably employed.

Note that, when the above-described feature values (1) to (8) are each calculated, the predetermined range of "the pixel number x × the number y of pixels" is taken out and the feature value is calculated to obtain a feature value convolved for a pixel at the center of "the pixel number x × the number y of pixels". Then, the feature value is calculated at each position while "the pixel number x × the number y of pixels" is moved in a metallographic image. Furthermore, when "the pixel number x × the number y of pixels" is positioned at an end (upper, lower, right, or left end) of the metallographic image, the feature value is calculated by applying a boundary condition or limiting the pixel numbers to the range of the center to the end. As for the boundary condition, the same feature value at the center of "the pixel number x × the number y of pixels" is applied to pixels outside the center of "the pixel number x × the number y of pixels". Alternatively, the feature value is calculated by applying extrapolation from the center toward the outside by using an extrapolation function such as a linear function, an exponential function, or a spline function.

<Phase classification process>

In the phase classification process, the phase classification unit 63 classifies the phase of the metallographic structure by using a learning-completed model (for example, a decision tree) generated in the model generation process (step S5) of the above-described learning method (step S14). Specifically, in the phase classification process, each feature value calculated in the feature value calculation process is input to a learning-completed model subjected to learning using feature values to which the labels of one or a plurality of phases designated for the metallographic structure in advance are allocated as inputs and the labels of the phases as outputs. Then, the phase label of any pixel corresponding to the input feature value is acquired to classify the phase of the metallographic structure in a metallographic image.

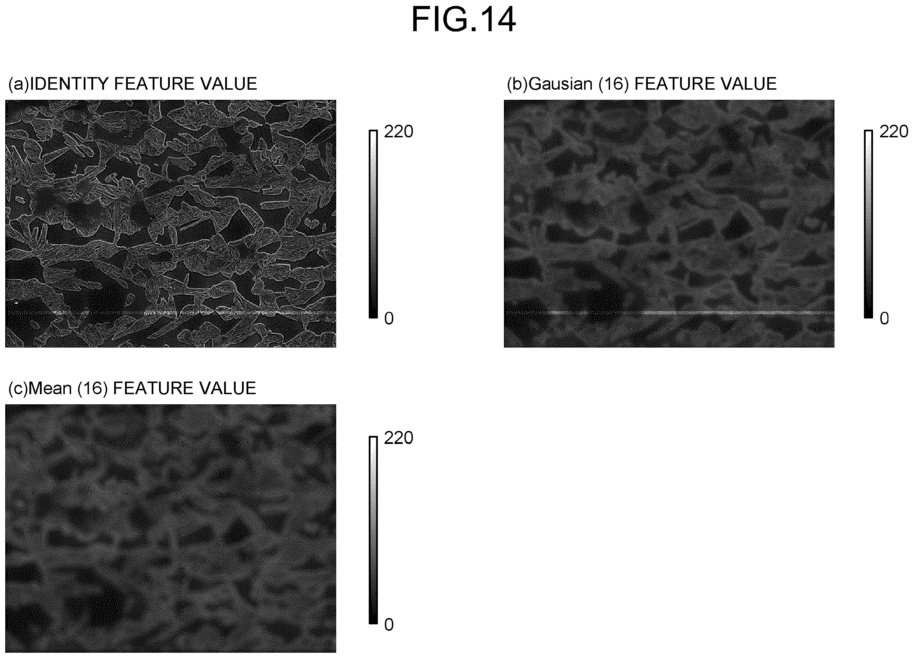

After the feature value calculation process is performed at step S13, each feature value calculation result may be output from the output unit 50. The outputting is not limited to a particular format, and may be performed in the format of a text file (for example, a set of numerical values) or an image file (for example, the histogram image in FIG. 12 or the metallographic image in FIG. 14). When each feature value calculation result is output in the format of a text file or an image file in this manner, the degree of classification with each feature value can be checked before phase classification is performed. Similarly, after the phase classification process is performed at step S14, a phase classification result may be output from the output unit 50. The outputting is not limited to a particular format, and may be performed in the format of a text file or an image file.

With the metallographic structure phase classification device and the metallographic structure phase classification method according to the first embodiment as described above, the important metallographic structure in a metallic material can be evaluated quantitatively and speedily by calculating a feature value of each phase from an image of the metallographic structure and learning the correspondence between the feature value and the phase.

Moreover, with the metallographic structure phase classification device and the metallographic structure phase classification method according to the first embodiment, image preprocessing is appropriately performed on a captured metallographic image, the phase of the metallographic image on which the image preprocessing is performed is designated, and a feature value of the designated phase is calculated and recorded. Then, appropriate binarization is repeatedly performed on the recorded feature value of each phase to learn a repeated binarization procedure. Then, phase classification of an optional metallographic image is automatically performed based on results of the phase learning, thereby accurately measuring the phase fraction of each phase.

(Metallographic structure phase classification method (second embodiment))

A metallographic structure phase classification method using the classification device 3 will be described with reference to FIG. 6. The metallographic structure phase classification method classifies two or more phases of the metallographic structure of one metallic material for a plurality of images captured of the metallographic structure of the metallic material. The metallographic structure phase classification method performs an image inputting process, an image preprocessing process, a feature value calculation process, a phase classification process, an error evaluation value calculation process, and a preprocessing adjustment process in the stated order. Note that, a specimen preparation process that the surface of a specimen is, for example, polished by a well-known method, and an image capturing process in which a metallographic image of a metallic material is captured by a well-known image capturing device such as an optical microscope or a scanning electron microscope may be performed before the image preprocessing process as necessary.

<Image inputting process>

In the image inputting process, the input unit 70 inputs an image of part of a specimen to the computation unit 90 (step S21). Specifically, in the image inputting process, one or a predetermined number of metallographic images are selected among a plurality of metallographic images captured of one metallic material and provided with specimen preparation as necessary, and each selected metallographic image is input to the computation unit 90.

<Image preprocessing process>

In the image preprocessing process, the image preprocessing unit 91 preprocesses the metallographic image (step S22). The contrast of the metallographic image (original image) input by the input unit 70 is often different among captured images due to difference in a specimen preparation method before image capturing, and the strength of a light source and contrast adjustment at image capturing, even in a case of image capturing of the same specimen by the same image capturing device. In addition, the luminance value of the metallographic image input by the input unit 70 is different among positions in some cases even in the same phase in the same image due to difference in the position of a light source in a case of an optical microscope, difference in the position of a detector in a case of an electron microscope, and the like. Such difference in the luminance value reduces learning efficiency and also causes error in phase classification. Thus, in the image preprocessing process, the metallographic image is preprocessed by adjusting the luminance value of each pixel of the metallographic image.

Similarly to the preprocessing process (step S2 in FIG. 4) of the above-described learning method, examples of metallographic image preprocessing include (1) identity processing, (2) background removal processing, and (3) histogram equalization processing. Note that, the contents of these pieces of processing are same as those of the preprocessing process of the above-described learning method, and thus description thereof is omitted. A plurality of the above-described pieces of processing (1) to (3) may be combined, or only one (for example, the identity processing) thereof may be performed as preprocessing. However, in a case of image capturing of a metallographic structure, the luminance value is different for each metallographic image or the luminance value is different among positions in the same image in many cases, and thus at least the background removal processing among the above-described pieces of processing (1) to (3) is preferably performed.

<Feature value calculation process>

In the feature value calculation process, the feature value calculation unit 92 calculates one or more feature values for each pixel of a metallographic image input in the image inputting process (step S23). In the feature value calculation process, similarly to the feature value calculation process (step S4 in FIG. 4) of the above-described learning method, one or more feature values among (1) the identity feature value, (2) the Mean feature value, (3) the Gaussian feature value, (4) the Median feature value, (5) the Max feature value, and (6) the Min feature value are calculated.

Furthermore, in the feature value calculation process, similarly to the feature value calculation process (step S4 in FIG. 4) of the above-described learning method, one or more feature values among the feature values (1) to (8), additionally including (7) the Derivative feature value and (8) the Derivative addition feature value may be calculated. Moreover, in the feature value calculation process, the feature values (7) and (8) may be calculated in addition to the feature values (1) to (6).

Since the above-described feature values (1) to (6) are computed for a large number of pixels of each phase, the same phase has different feature values and histograms of the feature values can be produced for each phase. The above-described feature values (1) to (6) may be all calculated or only some feature values may be calculated. Furthermore, a feature value obtained through a combination of the feature value computations may be added, and a feature value not described above may be added as necessary. Selection thereof is preferably made by the operator so that learning accuracy in a learning method improves, and a feature value that largely differs among phases is preferably employed.

Furthermore, since, similarly to the above-described feature values (1) to (6), the feature values (7) and (8) are computed for a large number of pixels of each phase, the same phase has different feature values and histograms of the feature values can be produced for each phase. The above-described feature values (1) to (8) may be all calculated or only some feature values may be calculated. Furthermore, a feature value obtained through a combination of the feature value computations may be added, and a feature value not described above may be added as necessary. Selection thereof is preferably made by the operator so that learning accuracy in a learning method improves, and a feature value that largely differs among phases is preferably employed.

Note that, when the above-described feature values (1) to (8) are each calculated, the predetermined range of "the pixel number x × the number y of pixels" is taken out and the feature value is calculated to obtain a feature value convolved for a pixel at the center of "the pixel number x × the number y of pixels". Then, the feature value is calculated at each position while "the pixel number x × the number y of pixels" is moved in a metallographic image. Furthermore, when "the pixel number x × the number y of pixels" is positioned at an end (upper, lower, right, or left end) of the metallographic image, the feature value is calculated by applying a boundary condition or limiting the pixel numbers to the range of the center to the end. As for the boundary condition, the same feature value at the center of "the pixel number x × the number y of pixels" is applied to pixels outside the center of "the pixel number x × the number y of pixels". Alternatively, the feature value is calculated by applying extrapolation from the center toward the outside by using an extrapolation function such as a linear function, an exponential function, or a spline function.

<Phase classification process>

In the phase classification process, the phase classification unit 93 classifies the phase of the metallographic structure by using a learning-completed model (for example, a decision tree) generated in the model generation process (step S5) of the above-described learning method (step S24). Specifically, in the phase classification process, each feature value calculated in the feature value calculation process is input to a learning-completed model subjected to learning using feature values to which the labels of one or a plurality of phases designated for the metallographic structure in advance are allocated as inputs and the labels of the phases as outputs. Then, the phase label of any pixel corresponding to the input feature value is acquired to classify the phase of the metallographic structure in a metallographic image.

After the feature value calculation process is performed at step S23, each feature value calculation result may be output from the output unit 80. The outputting is not limited to a particular format, and may be performed in the format of a text file (for example, a set of numerical values) or an image file (for example, the histogram image in FIG. 12 or the metallographic image in FIG. 14). When each feature value calculation result is output in the format of a text file or an image file in this manner, the degree of classification with each feature value can be checked before phase classification is performed. Similarly, after the phase classification process is performed at step S24, a phase classification result may be output from the output unit 80. The outputting is not limited to a particular format, and may be performed in the format of a text file or an image file.

<Error evaluation value calculation process>

In the error evaluation value calculation process, the error evaluation value calculation unit 94 calculates an error evaluation value by performing, for each pixel, true/false determination of each phase of the metallographic structure classified in the phase classification process (step S25).

<Preprocessing adjustment process>

In the preprocessing adjustment process, preprocessing conditions (preprocessing parameters) of the image preprocessing unit 91 are changed so that the error evaluation value calculated in the error evaluation value calculation process decreases, and the preprocessing conditions are adjusted by repeating the feature value calculation process, the phase classification process, and the error evaluation value calculation process.

In the preprocessing adjustment process, first, the preprocessing adjustment unit 95 determines whether the phase classification accuracy is equal to or higher than a predetermined threshold value based on the error evaluation value calculated in the error evaluation value calculation process (step S26). When it is determined that the phase classification accuracy is equal to or higher than the predetermined threshold value (Yes at step S26), the preprocessing conditions are fixed and other images of the specimen, in other words, a plurality of images captured of the metallographic structure of the metallic material are input (step S27). Then, the image preprocessing process (step S28), the feature value calculation process (step S29), and the phase classification process (step S30) are performed for all images.

When it is determined that the phase classification accuracy is lower than the predetermined threshold value (No at step S26), the preprocessing adjustment unit 95 changes the preprocessing conditions (step S31) and returns to step S22. Then, the image preprocessing process (step S22), the feature value calculation process (step S23), the phase classification process (step S24), and the error evaluation value calculation process (step S25) are performed until it is determined that the phase classification accuracy is equal to or higher than the predetermined threshold value at step S26.

The classification accuracy degrades in some cases when a metallographic image of a metallic material is temporarily captured, a specimen is produced, phase classification is learned, and then phase classification is performed on a metallographic image of a metallic material of the same steel kind or the same system. This is because, for example, the contrast of a captured image potentially changes even with the same kind of steel and the same system due to difference in etching, and image capturing, and other conditions of a specimen.

For example, the contrast of a metallographic image can be adjusted to some extent in the above-described image preprocessing process (step S22), whereas the preprocessing conditions (preprocessing parameters) include a changeable parameter, as well. Thus, improvement of the phase classification accuracy can be expected by adjusting the preprocessing conditions in accordance with a material.

Thus, the above-described processing at steps S21 to S25 is performed when a large number of metallographic images are collected for the same specimen and phase classification is performed. Specifically, one or a predetermined number of metallographic images are selected among a plurality of metallographic images and input (step S21), and the image preprocessing (step S22), the feature value calculation (step S23), and phase classification of the metallographic structure using a learning-completed model subjected to learning in advance (step S24) are performed. Then, evaluation of a result of the processing (in other words, the error evaluation value calculation (step S25)) is performed.

At step S25 described above, the result of phase classification at step S24 is compared with, for example, a result of phase classification by a human. In this case, the result of phase classification by a human is set as true, and true/false determination of each pixel for the phase classification result obtained at step S24 is performed to quantify error. For example, a true pixel is set as "0" and a false pixel is set as "1", and the sum of these values is calculated to obtain an error evaluation value.

Then, at step S31 described above, a luminance adjustment parameter as a preprocessing condition is changed so that the error evaluation value calculated at step S25 decreases and that the classification accuracy becomes equal to or higher than the predetermined threshold value (refer to step S26).

Examples of preprocessing parameters changed at step S31 include a width for calculating a mean luminance value with which background removal is performed. Furthermore, it is unknown which mean and standard deviation are desirable for homogenizing a histogram of the luminance value of a metallographic image, and thus it is considered that the values of the mean and the standard deviation as references are searched in a similar manner and that the histogram of the luminance value is changed. For the search for these parameters and the like, a typical optimization method can be employed.

After the preprocessing conditions are optimized based on phase classification results of one or a predetermined number of metallographic images, phase classification of the metallographic structure is performed for another metallographic image of the same specimen as described above at steps S27 to S30. In this case, the preprocessing conditions may be optimized basically with one metallographic image as a sample, but further improvement of the classification accuracy is expected by using, as a sample, a metallographic image within several % of the entire evaluation image.

With the metallographic structure phase classification device and the metallographic structure phase classification method according to the second embodiment as described above, the luminance level of a metallographic image can be optimized even for different metallic materials and thus phase classification of the metallographic image can be highly accurately performed.

(Metallic material property prediction device)

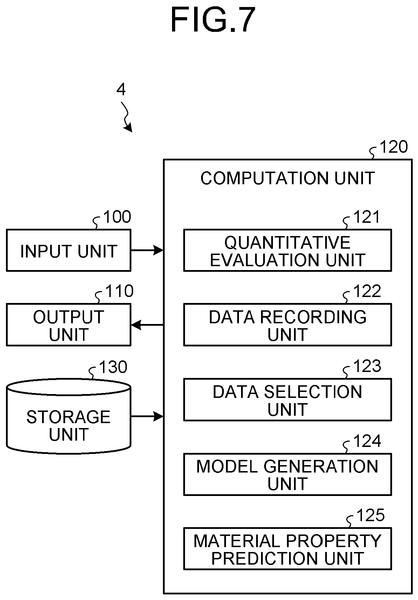

A metallic material property prediction device (hereinafter referred to as a "material property prediction device") 4 will be described with reference to FIG. 7. The material property prediction device 4 includes an input unit 100, an output unit 110, a computation unit 120, and a storage unit 130.

The input unit 100 is an input unit that inputs, to the computation unit 120, an image (hereinafter referred to as a "classification image") for which the phase of the metallographic structure is classified. This classification image is, for example, an image for which the phase of the metallographic structure is classified by the above-described metallographic structure phase classification method, or an image for which the phase of the metallographic structure is classified by another method such as luminance value binarization.

The output unit 110 is an output unit that outputs results of computation by the computation unit 120. The output unit 110 is configured as, for example, a display, a printer, or a smartphone. The output unit 110 outputs, for example, a quantitative evaluation value of the metallographic structure, which is calculated by a quantitative evaluation unit 121, data recorded in a database in the storage unit 130, and a result (material properties of a metallic material) of prediction by a material property prediction unit 125. Note that, the outputting by the output unit 110 is not limited to a particular format, and may be performed in, for example, the format of data such as a text file or an image file or the format of projection to an output device.

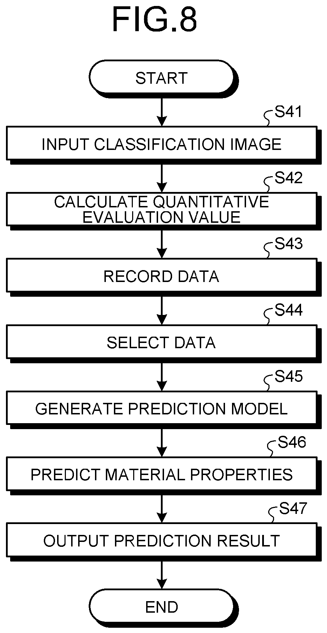

Similarly to the computation units 30, 60, and 90, the computation unit 120 is implemented with a processor made of a CPU or the like, and a memory made of a RAM, a ROM, or the like. The computation unit 120 loads a computer program onto a work area of the main storage unit, executes the computer program, and controls each component or the like through execution of the computer program, thereby achieving a function that matches a predetermined purpose. In addition, the computation unit 120 functions as the quantitative evaluation unit 121, a data recording unit 122, a data selection unit 123, a model generation unit 124, and the material property prediction unit 125 through the above-described computer program execution. Note that, each component will be described later in detail (refer to FIG. 8).

The storage unit 130 is configured as a recording medium such as an erasable programmable ROM (EPROM), a hard disk drive (HDD), a solid state drive (SSD), or a removable media. Examples of the removable media include disk recording media such as a universal serial bus (USB) memory, a compact disc (CD), a digital versatile disc (DVD), and a Blu-ray (registered trademark) disc (BD). In addition, the storage unit 130 can store an operating system (OS), various computer programs, various tables, various databases, and the like.

The storage unit 130 stores, for example, a database in which predetermined data is recorded and a prediction model (learning-completed model) generated by the model generation unit 124. The above-described database records, for example, quantitative evaluation values of the metallographic structure, which are calculated by the quantitative evaluation unit 121, material property values (steel plate data) of metallic materials, which are obtained by machine test or the like in advance, component composition of metallic materials, and metallic materials.

(Metallic material property prediction method)

The metallographic structure phase learning method using the material property prediction device 4 will be described with reference to FIG. 8. After the above-described metallographic structure phase classification method is performed, the metallographic structure phase learning method is performed by using a classification result (classification image) thus obtained. The metallographic structure phase learning method performs an image inputting process, a quantitative evaluation process, a data recording process, a data selection process, a model generation process, a material property prediction process, and a prediction result outputting process in the stated order.

<Image inputting process>

In the image inputting process, the input unit 100 inputs a classification image to the computation unit 120 (step S41).

<Quantitative evaluation process>

In the quantitative evaluation process, the quantitative evaluation unit 121 calculates a quantitative evaluation value of a metallographic structure by quantitatively evaluating each phase included in the classification image (step S42). In the quantitative evaluation process, for example, quantitative evaluation values described below in (1) to (5) are calculated.

(1) Area ratio

The area of a classified phase is determined to calculate the area ratio of the phase.

(2) Major radius, minor radius, and aspect ratio

The shape of each grain of a classified phase is approximated to an ellipse to calculate the major radius, minor radius, or aspect ratio of an ellipse body.

(3) Feret diameter

A straight line is drawn from the interface of each grain of a classified phase to calculate a Feret diameter with which the length of the straight line is maximum.

(4) Average diameter

The area of each grain of a classified phase is determined to derive the average diameter of the grain as the square root of the area.

(5) Circularity

The area and perimeter of each grain of a classified phase are determined to calculate the circularity of the grain by Expression (4) below. Note that, the circularity is 1.0 when the grain is an exact circle, and the circularity decreases from 1.0 as the grain is more dissimilar to the shape of an exact circle.

$$C = 4\pi \frac{S}{P^2} \tag{4}$$

**where C is the circularity,**
**S is the area, and**
**$P$ is the perimeter.**

Since the above-described quantitative evaluation values (2) to (5) are calculated for each grain, a plurality of numerical values can be obtained for one metallographic image and a histogram of each quantitative evaluation value can be produced.

<Data recording process>

In the data recording process, the data recording unit 122 records each quantitative evaluation value of a metallographic structure, which is calculated in the quantitative evaluation process, in the database in the storage unit 130 (step S43).

<Data selection process>

In the data selection process, the data selection unit 123 selects (extracts) data to be used for prediction of the material properties of a metallic material among data of the quantitative evaluation values of the metallographic structure, which are recorded in the database, and the material properties of metallic materials (step S44).

Note that, since the quantitative evaluation values are calculated for each grain, a plurality of numerical values are obtained for one metallographic image in some cases. For example, in a case of the average diameter as the quantitative evaluation value (4), one average diameter is obtained for each grain, and thus a plurality of numerical values are obtained for one image. A mean value may be calculated from information of the plurality of numerical values and only the mean value may be used for material property prediction, or a standard deviation may be used for material property prediction. Furthermore, a quantitative evaluation value with which the accuracy of material property prediction is high in a prediction model generation process to be described later is desirably selected in the data selection process. Thus, for example, when the prediction accuracy is low in the material property prediction process to be described later, it is preferable to return to the present process and perform data selection again. Note that, component composition of metallic materials and thermal treatment conditions may be input in addition to the quantitative evaluation value of the metallographic structure.

<Prediction model generation process>

In the prediction model generation process, the model generation unit 124 generates, by using the data selected in the data selection process, a prediction model for predicting the material properties of a metallic material (step S45). Specifically, in the prediction model generation process, a prediction model for predicting material properties is generated by using the quantitative evaluation value selected in the data selection process, component composition of metallic materials, thermal treatment conditions, and the like. The prediction model is generated by also using data of the material properties of metallic materials, which is selected in the data selection process.

The prediction model may be generated by using a model of neural network, support vector regression, Gaussian process regression, or the like and may be generated as a simple regression expression such as a linear regression. Moreover, in the prediction model generation, it is preferable to perform material property prediction by using a plurality of prediction models and employ a prediction model having the highest prediction accuracy.

The accuracy of material property prediction is evaluated by checking the degree of matching between data of a measured value and data of a predicted value based on, for example, a two-dimensional graph of the measured value on an X axis and the predicted value on a Y axis or is evaluated based on, for example, a parameter obtained by dividing the sum of prediction errors of the data by the number of pieces of data. Furthermore, the accuracy of material property prediction is preferably evaluated through, for example, a procedure as described below. First, data selected from a database is divided into data (training data) used for parameter fitting in the prediction model and data (test data) not used for fitting. Then, the accuracy of material property prediction is evaluated based on the degree of matching between a predicted value in the test data and the actual value. Note that, the division of data selected from a database into training data and test data may be selectively performed by the operator or may be determined at random by using a random number or the like after the ratio of training data and test data is determined.

<Material property prediction process>

In the material property prediction process, the material property prediction unit 125 predicts the material properties of a metallic material by using the prediction model generated in the prediction model generation process (step S46). Specifically, in the material property prediction process, the material properties of a metallic material are predicted by inputting the quantitative evaluation value selected in the data selection process into the prediction model generated in the prediction model generation process.

<Prediction result outputting process>

In the prediction result outputting process, the output unit 110 outputs a result of the prediction in the material property prediction process, in other words, the material properties of a metallic material (step S47).

With a conventional method, it has been difficult to perform efficient phase classification of the metallographic structure and quantitative evaluation of the metallographic structure using an image subjected to phase classification, and thus it has been difficult to perform accurate material property prediction based on a metallographic image. However, with the metallic material property prediction method and the metallic material property prediction device according to the present embodiment, quantitative evaluation can be efficiently performed based on results of phase classification of the metallographic structure. Thus, the material properties of a metallic material can be accurately predicted by deriving the correlation between a quantitative evaluation value and the material properties of the metallic material. This allows understanding of the material properties of the metallic material simultaneously with visual recognition of an image of a metallographic structure, thereby improving the efficiency of metallic material (for example, steel plate) development.

Moreover, with the metallic material property prediction method and the metallic material property prediction device according to the present embodiment, quantitative evaluation of a metallographic structure can be efficiently performed through efficient metallographic image segmentation, unlike conventional cases. In addition, the material properties of a captured metallographic image can be accurately predicted by using an indicator thus quantified.

[Examples]

(Example 1)

Example 1 of the metallographic structure phase learning method and the metallographic structure phase classification method according to the present invention will be described below with reference to FIGS. 9 to 16. In the present example, the specimen preparation process, the image capturing process, the image inputting process, the image preprocessing process, the phase designation process, the feature value calculation process, and the model generation process were performed as the metallographic structure phase learning method. In addition, the image inputting process, the feature value calculation process, and the phase classification process were performed as the metallographic structure phase classification method.

First, a DP steel plate (steel plate made of ferrite and martensite phases) was subjected to mirror polishing and Nital etching (the specimen preparation process), and the metallographic structure was observed by using a scanning electron microscope (the image capturing process). In FIG. 9, (a) illustrates a metallographic image (original image) captured by the scanning electron microscope, and (b) illustrates a line profile of the luminance value at a central part (the position of line L1) of the metallographic image.

Subsequently, the metallographic image was input to the learning device 1 (the image inputting process), and the image preprocessing process was performed. In FIG. 10, (a) illustrates the metallographic image after background removal was performed in the image preprocessing process, and (b) illustrates a line profile of the luminance value at a central part (the position of line L2) of the metallographic image. In the image preprocessing process, the background removal was performed by calculating the minimum value of the luminance value for each 50 pixels.

Subsequently, the phase designation process of designating ferrite and martensite phases was performed for the metallographic image provided with preprocessing of the background removal. FIG. 11 illustrates the ferrite and martensite phases designated in the phase designation process. In the figure, each region surrounded by a solid line is a ferrite phase, and each region surrounded by a dashed line is a martensite phase. In the present example, three ferrite phases and four martensite phases were designated, but the number of designated phases may be determined in accordance with machine specifications and the like.

Subsequently, feature values of the ferrite and martensite phases were calculated (the feature value calculation process). In the feature value calculation process, the identity feature value, and the Mean feature values, the Gaussian feature values, the Median feature values, the Max feature values, and the Min feature values of four pixels, eight pixels, and 16 pixels were calculated for each of the ferrite and martensite phases. In FIG. 12, (a), (b), and (c) illustrate a histogram representing the identity feature value, a histogram representing the Gaussian feature value calculated for the size of four pixels, and a histogram representing the Mean feature value calculated for the size of four pixels, respectively, among the 16 feature values in total. Note that, the number in parentheses of each feature value in (b) and (c) of FIG. 12 indicates a pixel size with which the feature value was calculated.

Note that, when a plurality of feature values are calculated in the feature value calculation process as illustrated in FIG. 12, a histogram of each feature value may be checked and a feature value to be learned later in the model generation process may be selected. In this case, a feature value for which the distance D between values at parts where the frequency is high in distributions of the feature value of the phases as illustrated in the figure is as large as possible is preferably selected as a feature value to be learned in the model generation process. The large distance D between values at parts where the frequency is high means that the feature values of the phases are likely to be separated from each other, and thus an accurate learning-completed model can be generated by learning a feature value for which the distance D is as large as possible. For example, in histograms (frequency distributions) obtained for feature values of phases, the difference (referred to as a distance D) between values of the feature value at which the frequency of the feature value is highest for the phases is normalized by the difference between the maximum and minimum values of the feature value. Then, it is thought that the normalized distance D is compared among feature values, and, for example, that a plurality of feature values having the larger normalized distance D are employed.

After the feature values were calculated in this manner, phase learning was performed by repeatedly performing binarization a plurality of times in the model generation process. In the present example, learning was performed by repeating binarization a plurality of times so that classification is achieved at the probability of 90% or higher for test data obtained by randomly extracting data from feature value distributions of the ferrite and martensite phases. FIG. 13 illustrates part of a decision tree generated as a result of the learning. Note that, the number in parentheses of each feature value set as a branching condition in FIG. 13 indicates a pixel size with which the feature value was calculated. In this learning example, a pixel is determined as ferrite when the Mean feature value calculated for the size of 16 pixels is smaller than 31.5 and the identity feature value is smaller than 12.5.

Subsequently, phase classification of a metallographic image was performed based on the learning result in FIG. 13. First, a metallographic image (refer to FIG. 10) provided with background removal in the image preprocessing process of the above-described learning method was input to the classification device 2 (the image inputting process), and feature values of the ferrite and martensite phases were calculated (the feature value calculation process).

In the feature value calculation process, the identity feature value, and the Mean feature values, the Gaussian feature values, the Median feature values, the Max feature values, and the Min feature values of four pixels, eight pixels, and 16 pixels were calculated for each of the ferrite and martensite phases. In FIG. 14, (a), (b), and (c) illustrate an image representing the identity feature value, an image representing the Gaussian feature value calculated for the size of 16 pixels, and an image representing the Mean feature value calculated for the size of 16 pixels, respectively, among the 16 feature values in total. Note that, the number in parentheses of each feature value in (b) and (c) of FIG. 14 indicates a pixel size with which the feature value was calculated.

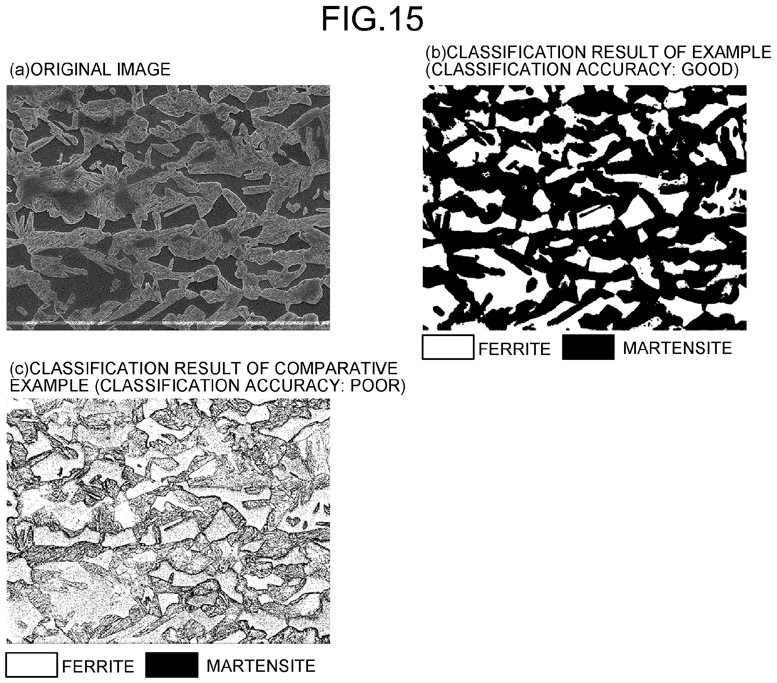

Subsequently, each feature value calculated in the feature value calculation process was classified by using a decision tree (branching diagram of the ferrite and martensite phases) generated by the learning device 1 described above. Results thereof are illustrated in FIG. 15. In FIG. 15, (a) is an original image before preprocessing, (b) is a classification result of the example, and (c) is a classification result of a comparative example. The classification result of the comparative example is a result obtained by classifying each feature value of the metallographic image by using a conventional binarization method.

In the classification result of the comparative example, as illustrated in (c) of FIG. 15, a large number of martensite phases are classified as a ferrite phase, and a large number of ferrite phases are classified as a martensite phase, which indicates that the classification accuracy is low. Thus, the phase fraction of ferrite and martensite phases cannot be accurately obtained even by determining the area of each white part and the area of each black part in this binarized image.

However, in the classification result of the example, as illustrated in (b) of FIG. 15, the accuracy is higher in the comparative example, and classification of ferrite and martensite phases can be far more efficiently performed than by a manual classification method that color-codes each phase by hand.

(Example 2)

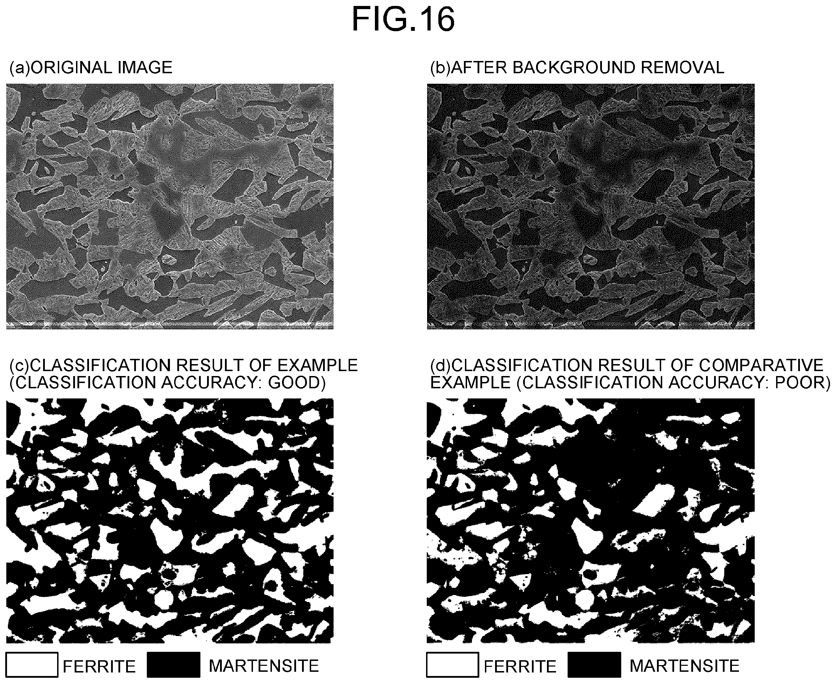

Example 2 of the metallographic structure phase classification method according to the present invention will be described with reference to FIG. 16. In the present example, feature values were calculated for each of a metallographic image provided with the image preprocessing process (background removal) and a metallographic image not provided with the image preprocessing process (background removal), and the phase of the metallographic structure was classified by using the learning result (for example, a decision tree) of Example 1 described above. In other words, in the present example, discussion was performed on difference in the phase classification accuracy depending on existence of the image preprocessing process.

First, a DP steel plate (steel plate made of ferrite and martensite phases) was subjected to mirror polishing and Nital etching (the specimen preparation process), and the metallographic structure was observed by using a scanning electron microscope (the image capturing process). In FIG. 16, (a) illustrates a metallographic image captured by the scanning electron microscope. Subsequently, the metallographic image was input to the classification device 2 (the image inputting process), and the image preprocessing process was performed. In FIG. 16, (b) illustrates the metallographic image after background removal was performed in the image preprocessing process.

Subsequently, feature values of the ferrite and martensite phases were calculated for the metallographic image provided with the background removal in the image preprocessing process. In the feature value calculation process, the identity feature value, and the Mean feature values, the Gaussian feature values, the Median feature values, the Max feature values, and the Min feature values of four pixels, eight pixels, and 16 pixels were calculated for each of the ferrite and martensite phases.

Subsequently, each feature value calculated in the feature value calculation process was classified by using a decision tree (branching diagram of the ferrite and martensite phases) generated in Example 1 described above. In FIG. 16, (c) is a classification result of the example, and (d) is a classification result of a comparative example. The classification result of the example is a result obtained by classifying the feature values of the metallographic image provided with the background removal, by using a decision tree subjected to learning with the feature values of the metallographic image provided with the background removal. The classification result of the comparative example is a result obtained by classifying the feature values of the metallographic image not provided with the background removal, by using a decision tree subjected to learning with the feature values of the metallographic image not provided with the background removal.

In the classification result of the example, as illustrated in (c) of FIG. 16, the classification accuracy is high, and classification of ferrite and martensite phases can be far more efficiently performed than by a manual classification method that color-codes each phase by hand.

However, in the classification result of the comparative example, as illustrated in (d) of FIG. 16, a large number of martensite phases nonetheless are classified as a ferrite phase, and a large number of ferrite phases nonetheless are classified as a martensite phase, which indicates that the classification accuracy is low. Thus, the phase fraction of ferrite and martensite phases cannot be accurately obtained even by determining the area of each white part and the area of each black part in this binarized image.

(Example 3)

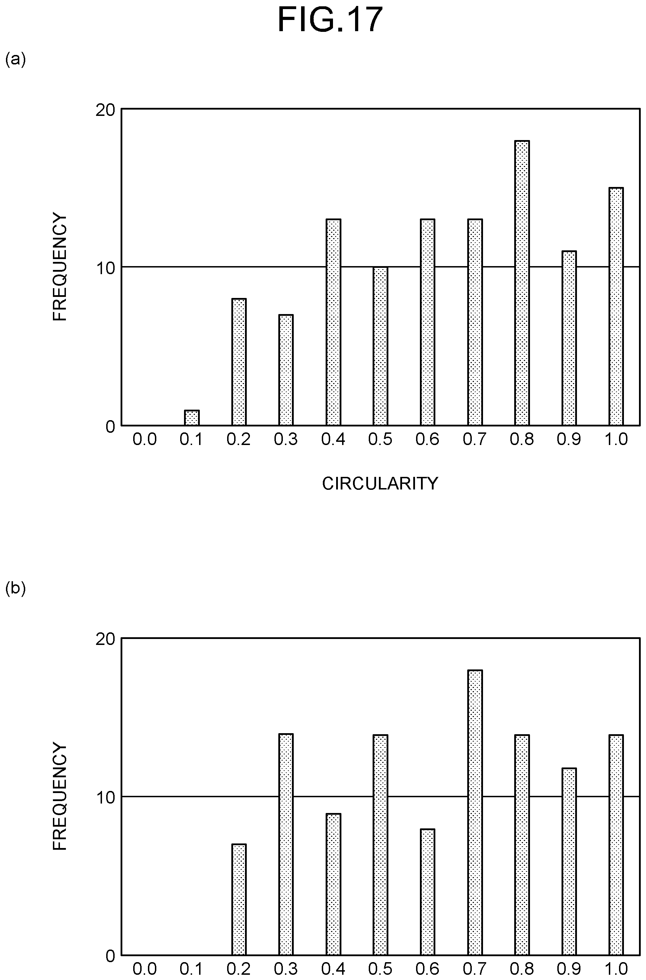

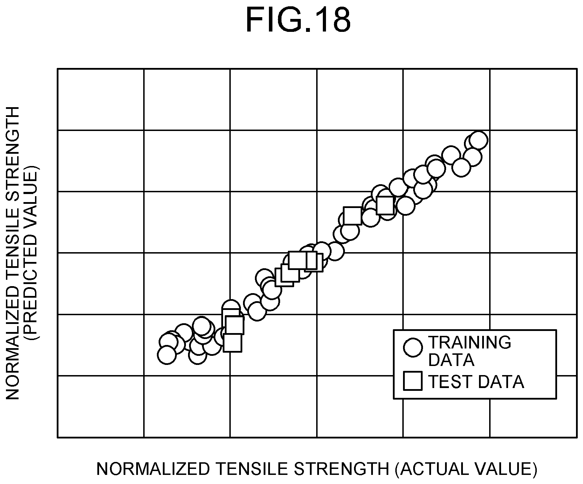

Example 3 of the metallic material property prediction method according to the present invention and the metallic material property prediction device will be described below with reference to FIGS. 17 and 18.

In the present example, first, quantitative evaluation of the metallographic structure was performed by using the classification result (refer to (b) of FIG. 15) of Example 1 and the classification result (refer to (c) of FIG. 16) of Example 2 described above (the quantitative evaluation process). In the evaluation, the area ratio and the histogram of the circularity were calculated for the ferrite and martensite phases. In FIG. 17, (a) illustrates a circularity histogram calculated for the ferrite phase from the classification result (refer to (b) of FIG. 15) of Example 1, and (b) illustrates a circularity histogram calculated for the ferrite phase from the classification result (refer to (c) of FIG. 16) of Example 2.

Subsequently, mean values of the area ratio and circularity values of the ferrite and martensite phases among quantitative evaluation values calculated as described above, and component composition of metallic materials other than the quantitative evaluation values were selected (the data selection process), and these pieces of data were used for material property prediction.

Subsequently, data of 100 kinds of steel were randomly extracted from a DP steel plate database including metallographic images, component composition of metallic materials, and tensile strength. Then, the same phase classification was performed for the extracted data, and thereafter, the mean values of the area ratio and circularity values of the ferrite and martensite phases described above were calculated.

Subsequently, a prediction model that predicts the tensile strength was generated from the above-described quantitative evaluation values and component composition of metallic materials (the prediction model generation process). Note that, in this example, the extracted data was randomly divided into training data and test data at the ratio of 9:1. In addition, a prediction model that predicts the tensile strength was generated by using a neural network model.

Subsequently, comparison between the actual value of the tensile strength and a predicted value was performed to verify the prediction accuracy of the prediction model. FIG. 18 illustrates a result of tensile strength prediction using the neural network model generated by the model generation unit. In the figure, the horizontal axis represents the actual value of the tensile strength normalized by using the mean value and standard deviation of the tensile strength extracted from a database. The vertical axis represents the predicted value of the tensile strength normalized by using the mean value and standard deviation of the tensile strength extracted from the database. In the figure, a circular plot point represents a prediction result of the tensile strength of a sample (training data) used for parameter adjustment of the neural network model. In addition, a rectangular plot point represents a prediction result of the tensile strength of a sample (test data) not used for the parameter adjustment.

As illustrated in FIG. 18, the accuracy of material property prediction is high for the training data and the test data, and the tensile strength is accurately predicted by using the mean values of the area ratio and circularity values of the ferrite and martensite phases and component composition of metallic materials. Accordingly, material properties can be accurately predicted based on metallographic images and component composition of metallic materials by using the method of the present invention, thereby improving the efficiency of steel plate development.

Although the above description is specifically made on the metallographic structure phase classification method, the metallographic structure phase classification device, the metallographic structure phase learning method, the metallographic structure phase learning device, the metallic material property prediction method, and the metallic material property prediction device according to the present invention with reference to the embodiments and the examples, the scope of the present invention is not limited to the description but should be widely interpreted based on description of the claims. In addition, for example, various changes and modifications based on the description are included in the scope of the present invention.

The metallographic structure phase learning method, the metallographic structure phase classification method, and the metallic material property prediction method according to the present invention may be each achieved through installation of a software application in which the method is implemented onto a typical commercially available computer. The typical commercially available computer is, for example, a computing unit including a CPU that executes commands of a computer program as a software application that achieves each function, a recording medium (such as a hard disk or a USB memory) in which the above-described software application and various kinds of data are recorded in a computer (or CPU)-readable form, a RAM onto which the above-described computer program is loaded, and a GPU as a processor specialized for image processing. Alternatively, each method may be achieved through installation of the software application on a cloud computer in a network instead of a commercially available computer.

Furthermore, the learning device 1, the classification devices 2 and 3, and the material property prediction device 4 are described as separate configurations as illustrated in FIGS. 1, 2, 3 and 7, but may be achieved as separate devices or may be achieved as one device. When the learning device 1, the classification devices 2 and 3, and the material property prediction device 4 are achieved as one device, the inputs unit 10, 40, 70, and 100 may be physically identical to one another, the output units 20, 50, 80, and 110 may be physically identical to one another, and the computation units 30, 60, 90, and 120 may be physically identical to one another.

Furthermore, in the present embodiment, description is performed on a duplex steel plate as an example, but the present invention is also applicable to a steel plate of three phases or more.

Reference Signs List

| | |
|---|---|
| 1 | learning device |
| 10 | input unit |
| 20 | output unit |
| 30 | computation unit |
| 31 | image preprocessing unit |
| 32 | phase designation unit |
| 33 | feature value calculation unit |
| 34 | model generation unit |
| 2 | classification device |
| 40 | input unit |
| 50 | output unit |
| 60 | computation unit |
| 61 | image preprocessing unit |
| 62 | feature value calculation unit |
| 63 | phase classification unit |
| 3 | classification device |
| 70 | input unit |
| 80 | output unit |
| 90 | computation unit |
| 91 | image preprocessing unit |
| 92 | feature value calculation unit |
| 93 | phase classification unit |
| 94 | error evaluation value calculation unit |
| 95 | preprocessing adjustment unit |
| 4 | material property prediction device |
| 100 | input unit |
| 110 | output unit |
| 120 | computation unit |
| 121 | quantitative evaluation unit |
| 122 | data recording unit |
| 123 | data selection unit |

124    model generation unit
125    material property prediction unit
130    storage unit

**Claims**

**1.** A metallographic structure phase classification method of classifying two or more phases of the metallographic structure of one metallic material for a plurality of images captured of the metallographic structure of the metallic material, the metallographic structure phase classification method comprising:

an image preprocessing step of selecting one or a predetermined number of images among the plurality of images and adjusting the luminance value of each pixel of each selected image;

a feature value calculation step of calculating one or more feature values for each pixel of the image preprocessed in the image preprocessing step;

a phase classification step of classifying the phase of the metallographic structure in the image by inputting each feature value calculated in the feature value calculation step to a learning-completed model subjected to learning using feature values to which labels of a plurality of phases of the metallographic structure are allocated as inputs and the labels of the phases as outputs and acquiring the label of the phase of a pixel corresponding to the input feature value;

an error evaluation value calculation step of calculating an error evaluation value by true/false determination, for each pixel, of each phase of the metallographic structure classified in the phase classification step; and

a preprocessing adjustment step of adjusting a preprocessing parameter of the image preprocessing step by changing the preprocessing parameter so that the error evaluation value calculated in the error evaluation value calculation step decreases and repeating the feature value calculation step, the phase classification step, and the error evaluation value calculation step,

wherein the preprocessing parameter adjusted in the preprocessing adjustment step is fixed and the image preprocessing step, the feature value calculation step, and the phase classification step are performed for all of a plurality of images captured of the metallographic structure of the metallic material.

**2.** The metallographic structure phase classification method according to claim 1, wherein the feature value calculation step calculates one or more feature values among

an identity feature value representing the luminance value of the image,

a Mean feature value representing a mean value of the luminance value in a predetermined range of the image,

a Gaussian feature value representing the mean value of the luminance value having a larger weight at a position closer to the center in the predetermined range of the image,

a Median feature value representing the center value of the luminance value in the predetermined range of the image,

a Max feature value representing the maximum value of the luminance value in the predetermined range of the image,

a Min feature value representing the minimum value of the luminance value in the predetermined range of the image,

a Derivative feature value representing a differential value of the luminance value of the image, and

a Derivative addition feature value obtained by adding the Derivative feature value.

**3.** The metallographic structure phase classification method according to claim 2, wherein the predetermined range is a range in which, among one or a plurality of phases designated for the metallographic structure in advance, a size smaller than 1/2 of a smaller crystal grain size is included and a size smaller than 1/2 of a larger crystal grain size is included.

**4.** The metallographic structure phase classification method according to any one of claims 1 to 3, wherein

the metallic material is a duplex steel plate, and

the phase classification step classifies ferrite and martensite phases.

**5.** The metallographic structure phase classification method according to any one of claims 1 to 4, wherein the learning-completed model is a machine learning model including a decision tree, a random forest, or a neural network.

6. A metallographic structure phase classification device comprising:

an image preprocessing unit configured to select one or a predetermined number of images among a plurality of images and adjusting the luminance value of each pixel of each selected image;

a feature value calculation unit configured to calculate one or more feature values for each pixel of an image captured of a metallographic structure of a metallic material;

a phase classification unit configured to classify the phase of the metallographic structure in the image by inputting each feature value calculated by the feature value calculation unit to a learning-completed model subjected to learning using feature values to which labels of one or a plurality of phases designated for the metallographic structure in advance are allocated as inputs and the labels of the phases as outputs and acquiring the label of the phase of a pixel corresponding to the input feature value; an image preprocessing unit configured to adjust the luminance value of each pixel of the image;

an error evaluation value unit configured to calculate an error evaluation value by true/false determination, for each pixel, of each phase of the metallographic structure; and

a preprocessing adjustment unit configured to adjust a preprocessing parameter of the image preprocessing unit by changing the preprocessing parameter so that the error evaluation value calculated by the error evaluation value unit decreases,

wherein the preprocessing parameter adjusted by the preprocessing adjustment unit is fixed and the image preprocessing step, the feature value calculation step, and the phase classification step are performed for all of a plurality of images captured of the metallographic structure of the metallic material.

7. A metallographic structure phase learning method for generating the learning-completed model used in the metallographic structure phase classification method according to any one of claims 1 to 5, the metallographic structure phase learning method comprising:

a phase designation step of allocating one of labels of one or a plurality of phases designated for the metallographic structure of a metallic material in advance to a pixel corresponding to the phase in an image captured of the metallographic structure;

a feature value calculation step of calculating one or more feature values for each of the pixels to which the labels of the phases are allocated; and

a model generation step of generating a learning-completed model through learning using the feature values to which the labels of the phases are allocated as inputs and the labels of the phases as outputs.

8. A metallographic structure phase learning device for generating the learning-completed model used in the metallographic structure phase classification device according to claim 6, the metallographic structure phase learning device comprising:

a phase designation unit configured to allocate one of labels of one or a plurality of phases designated for the metallographic structure of a metallic material in advance to a pixel corresponding to the phase in an image captured of the metallographic structure;

a feature value calculation unit configured to calculate one or more feature values for each of the pixels to which the labels of the phases are allocated; and

a model generation unit configured to generate a learning-completed model through learning using the feature values to which the labels of the phases are allocated as inputs and the labels of the phases as outputs.

9. A metallic material property prediction method of predicting material properties of a metallic material, the metallic material property prediction method comprising, after the metallographic structure phase classification method according to any one of claims 1 to 5:

a quantitative evaluation step of calculating a quantitative evaluation value of a metallographic structure by calculating the size, area ratio, or shape of each classified phase;

a data selection step of selecting data to be used to predict the material properties of the metallic material among the quantitative evaluation value and the material properties of the metallic material prepared in advance;

a model generation step of generating, by using the selected data, a prediction model that predicts the material properties of the metallic material; and

a material property prediction step of predicting the material properties of the metallic material by using the generated prediction model.

10. A metallic material property prediction device configured to predict material properties of a metallic material, the metallic material property prediction device comprising:

an input unit configured to input an image for which phase classification of a metallographic structure is performed by the metallographic structure phase classification device of claim 6;
a quantitative evaluation unit configured to calculate a quantitative evaluation value of the metallographic structure by calculating the size, area ratio, or shape of each classified phase;
a data recording unit configured to record the quantitative evaluation value in a database;
a data selection unit configured to select data to be used to predict the material properties of the metallic material among the quantitative evaluation value recorded in the database and the material properties of the metallic material;
a model generation unit configured to generate, by using the selected data, a prediction model that predicts the material properties of the metallic material;
a material property prediction unit configured to predict the material properties of the metallic material by using the generated prediction model; and
an output unit configured to output the predicted material properties of the metallic material.

**Patentansprüche**

1. Verfahren zur Klassifizierung von Phasen einer metallographischen Struktur zum Klassifizieren von zwei oder mehr Phasen der metallographischen Struktur eines metallischen Materials für eine Vielzahl von Bildern, die von der metallographischen Struktur des metallischen Materials aufgenommen wurden, umfassend:

einen Bildvorverarbeitungsschritt zum Auswählen eines oder einer vorgegebenen Anzahl von Bildern aus der Vielzahl von Bildern und zum Anpassen des Luminanzwerts jedes Pixels jedes ausgewählten Bildes;
einen Merkmalswertberechnungsschritt zum Berechnen eines oder mehrerer Merkmalswerte für jedes Pixel des im Bildvorverarbeitungsschritt vorverarbeiteten Bildes;
einen Phasenklassifizierungsschritt zum Klassifizieren der Phase der metallographischen Struktur in dem Bild, indem jeder im Merkmalswertberechnungsschritt berechnete Merkmalswert einem lernabgeschlossenen Modell zugeführt wird, das unter Verwendung von Merkmalswerten gelernt hat,
wobei den Merkmalswerten Labels einer Vielzahl von Phasen der metallographischen Struktur als Eingaben und die Labels der Phasen als Ausgaben zugewiesen werden und das Label der Phase eines Pixels, das dem eingegebenen Merkmalswert entspricht, erfasst wird;
einen Fehlerbewertungswertberechnungsschritt zum Berechnen eines Fehlerbewertungswerts durch Wahr-/Falsch-Bestimmung für jedes Pixel jeder im Phasenklassifizierungsschritt klassifizierten Phase der metallographischen Struktur; und
einen Vorverarbeitungseinstellschritt zum Einstellen eines Vorverarbeitungsparameters des Bildvorverarbeitungsschritts, indem der Vorverarbeitungsparameter so geändert wird, dass der im Fehlerbewertungswertberechnungsschritt berechnete Fehlerbewertungswert abnimmt, und durch Wiederholen des Merkmalswertberechnungsschritts, des Phasenklassifizierungsschritts und des Fehlerbewertungswertberechnungsschritts,
wobei der im Vorverarbeitungseinstellschritt angepasste Vorverarbeitungsparameter fixiert wird und der Bildvorverarbeitungsschritt, der Merkmalswertberechnungsschritt und der Phasenklassifizierungsschritt für alle Bilder einer Vielzahl von Bildern ausgeführt werden, die von der metallographischen Struktur des metallischen Materials aufgenommen wurden.

2. Verfahren zur Klassifizierung von Phasen einer metallographischen Struktur nach Anspruch 1, wobei der Merkmalswertberechnungsschritt einen oder mehrere der folgenden Merkmalswerte berechnet:

einen Identity-Merkmalswert, der den Luminanzwert des Bildes repräsentiert,
einen Mean-Merkmalswert, der einen Mittelwert des Luminanzwerts in einem vorgegebenen Bereich des Bildes repräsentiert,
einen Gaussian-Merkmalswert, der den Mittelwert des Luminanzwerts mit einem größeren Gewicht an einer Position repräsentiert, die näher an der Mitte in dem vorgegebenen Bereich des Bildes liegt,
einen Median-Merkmalswert, der den Zentralwert des Luminanzwerts in dem vorgegebenen Bereich des Bildes repräsentiert,
einen Max-Merkmalswert, der den Maximalwert des Luminanzwerts in dem vorgegebenen Bereich des Bildes repräsentiert,

einen Min-Merkmalswert, der den Minimalwert des Luminanzwerts in dem vorgegebenen Bereich des Bildes repräsentiert,
einen Derivative-Merkmalswert, der einen Differentialwert des Luminanzwerts des Bildes repräsentiert, und
einen Derivative-Addition-Merkmalswert, der durch Addieren des Derivative-Merkmalswerts erhalten wird.

3. Verfahren zur Klassifizierung von Phasen einer metallographischen Struktur nach Anspruch 2, wobei der vorgegebene Bereich ein Bereich ist, in dem von einer oder einer Vielzahl von Phasen, die für die metallographische Struktur im Voraus festgelegt sind, eine Größe enthalten ist, die kleiner als 1/2 einer kleineren Kristallkorngröße ist, und eine Größe enthalten ist, die kleiner als 1/2 einer größeren Kristallkorngröße ist.

4. Verfahren zur Klassifizierung von Phasen einer metallographischen Struktur nach einem der Ansprüche 1 bis 3, wobei

das metallische Material eine Duplexstahlplatte ist, und
der Phasenklassifizierungsschritt Ferrit- und Martensitphasen klassifiziert.

5. Verfahren zur Klassifizierung von Phasen einer metallographischen Struktur nach einem der Ansprüche 1 bis 4, wobei das lernabgeschlossene Modell ein maschinelles Lernmodell ist, das einen Entscheidungsbaum, einen Random Forest oder ein neuronales Netzwerk umfasst.

6. Vorrichtung zur Klassifizierung von Phasen einer metallographischen Struktur, umfassend:

eine Bildvorverarbeitungseinheit, die ausgebildet ist, um ein oder eine vorgegebene Anzahl von Bildern aus einer Vielzahl von Bildern auszuwählen und den Luminanzwert jedes Pixels jedes ausgewählten Bildes anzupassen;
eine Merkmalswertberechnungseinheit, die ausgebildet ist, um einen oder mehrere Merkmalswerte für jedes Pixel eines Bildes zu berechnen, das von einer metallographischen Struktur eines metallischen Materials aufgenommen wurde;
eine Phasenklassifizierungseinheit, die ausgebildet ist, um die Phase der metallographischen Struktur in dem Bild zu klassifizieren, indem jeder von der Merkmalswertberechnungseinheit berechnete Merkmalswert in ein lernabgeschlossenes Modell eingegeben wird, das unter Verwendung von Merkmalswerten gelernt hat,

wobei den Merkmalswerten Labels einer oder einer Vielzahl von Phasen, die für die metallographische Struktur im Voraus festgelegt sind, als Eingaben und die Labels der Phasen als Ausgaben zugewiesen werden und das Label der Phase eines Pixels, das dem eingegebenen Merkmalswert entspricht, erfasst wird; eine Bildvorverarbeitungseinheit, die ausgebildet ist, den Luminanzwert jedes Pixels des Bildes anzupassen;
eine Fehlerbewertungswerteinheit, die ausgebildet ist, einen Fehlerbewertungswert durch Wahr/Falsch-Bestimmung für jedes Pixel jeder Phase der metallographischen Struktur zu berechnen; und

eine Vorverarbeitungseinstelleinheit, die ausgebildet ist, einen Vorverarbeitungsparameter der Bildvorverarbeitungseinheit einzustellen, indem der Vorverarbeitungsparameter so geändert wird, dass der von der Fehlerbewertungswerteinheit berechnete Fehlerbewertungswert abnimmt,
wobei der von der Vorverarbeitungseinstelleinheit angepasste Vorverarbeitungsparameter fixiert wird und der Bildvorverarbeitungsschritt, der Merkmalswertberechnungsschritt und der Phasenklassifizierungsschritt für alle Bilder einer Vielzahl von Bildern ausgeführt werden, die von der metallographischen Struktur des metallischen Materials aufgenommen wurden.

7. Verfahren zum Lernen der Phasen einer metallographischen Struktur zum Erzeugen des in dem Verfahren zur Klassifizierung von Phasen einer metallographischen Struktur nach einem der Ansprüche 1 bis 5 verwendeten lernabgeschlossenen Modells, wobei das Verfahren zum Lernen der Phasen einer metallographischen Struktur umfasst:

einen Schritt zur Phasenbezeichnung, bei dem einem Pixel, das einer Phase in einem von der metallographischen Struktur eines metallischen Materials aufgenommenen Bild entspricht, eines der Labels einer oder einer Vielzahl von Phasen zugewiesen wird, die für die metallographische Struktur im Voraus festgelegt sind;
einen Merkmalswertberechnungsschritt zum Berechnen eines oder mehrerer Merkmalswerte für jedes der Pixel, denen die Labels der Phasen zugewiesen sind; und
einen Modellgenerierungsschritt zum Erzeugen eines lernabgeschlossenen Modells durch Lernen unter Verwendung der Merkmalswerte, denen die Labels der Phasen zugewiesen sind, als Eingaben und der Labels der Phasen als Ausgaben.

8. Vorrichtung zum Lernen der Phasen einer metallographischen Struktur zum Erzeugen des in der Vorrichtung zur Klassifizierung von Phasen einer metallographischen Struktur nach Anspruch 6 verwendeten lernabgeschlossenen Modells, wobei die Vorrichtung zum Lernen der Phasen einer metallographischen Struktur umfasst:

eine Phasenbezeichnungseinheit, die ausgebildet ist, einem Pixel, das einer Phase in einem von der metallographischen Struktur eines metallischen Materials aufgenommenen Bild entspricht, eines der Labels einer oder einer Vielzahl von Phasen zuzuweisen, die für die metallographische Struktur im Voraus festgelegt sind;

eine Merkmalswertberechnungseinheit, die ausgebildet ist, einen oder mehrere Merkmalswerte für jedes der Pixel zu berechnen, denen die Labels der Phasen zugewiesen sind; und

eine Modellgenerierungseinheit, die ausgebildet ist, ein lernabgeschlossenes Modell durch Lernen unter Verwendung der Merkmalswerte, denen die Labels der Phasen zugewiesen sind, als Eingaben und der Labels der Phasen als Ausgaben zu erzeugen.

9. Verfahren zur Vorhersage einer Eigenschaft eines metallischen Materials zum Vorhersagen von Materialeigenschaften eines metallischen Materials, wobei das Verfahren zur Vorhersage einer Eigenschaft eines metallischen Materials nach dem Verfahren zur Klassifizierung von Phasen einer metallographischen Struktur nach einem der Ansprüche 1 bis 5 Folgendes umfasst:

einen Schritt zur quantitativen Bewertung zum Berechnen eines quantitativen Bewertungswerts einer metallographischen Struktur durch Berechnen der Größe, des Flächenverhältnisses oder der Form jeder klassifizierten Phase;

einen Datenauswahlschritt zum Auswählen von Daten, die zur Vorhersage der Materialeigenschaften des metallischen Materials verwendet werden sollen, aus dem quantitativen Bewertungswert und den im Voraus bereitgestellten Materialeigenschaften des metallischen Materials;

einen Modellgenerierungsschritt zum Erzeugen eines Vorhersagemodells unter Verwendung der ausgewählten Daten, das die Materialeigenschaften des metallischen Materials vorhersagt; und

einen Materialeigenschaftsvorhersageschritt zum Vorhersagen der Materialeigenschaften des metallischen Materials unter Verwendung des erzeugten Vorhersagemodells.

10. Vorrichtung zur Vorhersage einer Eigenschaft eines metallischen Materials, die zum Vorhersagen von Materialeigenschaften eines metallischen Materials ausgebildet ist, wobei die Vorrichtung zur Vorhersage einer Eigenschaft eines metallischen Materials umfasst:

eine Eingabeeinheit, die ausgebildet ist, ein Bild einzugeben, für das eine Phasenklassifizierung einer metallographischen Struktur durch die Vorrichtung zur Klassifizierung von Phasen einer metallographischen Struktur nach Anspruch 6 durchgeführt wird;

eine Einheit zur quantitativen Bewertung, die ausgebildet ist, einen quantitativen Bewertungswert der metallographischen Struktur durch Berechnen der Größe, des Flächenverhältnisses oder der Form jeder klassifizierten Phase zu berechnen;

eine Datenaufzeichnungseinheit, die ausgebildet ist, den quantitativen Bewertungswert in einer Datenbank aufzuzeichnen;

eine Datenauswahleinheit, die ausgebildet ist, Daten auszuwählen, die zur Vorhersage der Materialeigenschaften des metallischen Materials verwendet werden sollen, aus dem in der Datenbank aufgezeichneten quantitativen Bewertungswert und den Materialeigenschaften des metallischen Materials;

eine Modellgenerierungseinheit, die ausgebildet ist, unter Verwendung der ausgewählten Daten ein Vorhersagemodell zu erzeugen, das die Materialeigenschaften des metallischen Materials vorhersagt;

eine Materialeigenschaftsvorhersageeinheit, die ausgebildet ist, die Materialeigenschaften des metallischen Materials unter Verwendung des erzeugten Vorhersagemodells vorherzusagen; und

eine Ausgabeeinheit, die ausgebildet ist, die vorhergesagten Materialeigenschaften des metallischen Materials auszugeben.

## Revendications

1. Procédé de classification de phase de structure métallographique consistant à classer deux phases ou plus de la structure métallographique d'un matériau métallique pour une pluralité d'images capturées de la structure métallographique du matériau métallique, le procédé de classification de phase de structure métallographique comprenant :

une étape de prétraitement d'image consistant à sélectionner une ou un nombre prédéterminé d'images parmi la pluralité d'images et à ajuster la valeur de luminance de chaque pixel de chaque image sélectionnée ;

une étape de calcul de valeur de caractéristique consistant à calculer une ou plusieurs valeurs de caractéristique pour chaque pixel de l'image prétraitée lors de l'étape de prétraitement d'image ;

une étape de classification de phase consistant à classer la phase de la structure métallographique dans l'image en entrant chaque valeur de caractéristique calculée lors de l'étape de calcul de valeur de caractéristique dans un modèle dont l'apprentissage est terminé ayant été soumis à un apprentissage en utilisant des valeurs de caractéristique auxquelles des libellés d'une pluralité de phases de la structure métallographique sont attribués en tant qu'entrées et les libellés des phases en tant que sorties, et en acquérant le libellé de la phase d'un pixel correspondant à la valeur de caractéristique d'entrée ;

une étape de calcul de valeur d'évaluation d'erreur consistant à calculer une valeur d'évaluation d'erreur par détermination vrai/faux, pour chaque pixel, de chaque phase de la structure métallographique classifiée lors de l'étape de classification de phase ; et

une étape d'ajustement de prétraitement consistant à ajuster un paramètre de prétraitement de l'étape de prétraitement d'image en modifiant le paramètre de prétraitement de telle sorte que la valeur d'évaluation d'erreur calculée lors de l'étape de calcul de valeur d'évaluation d'erreur diminue et en répétant l'étape de calcul de valeur de caractéristique, l'étape de classification de phase et l'étape de calcul de valeur d'évaluation d'erreur,

dans lequel le paramètre de prétraitement ajusté lors de l'étape d'ajustement de prétraitement est fixe et l'étape de prétraitement d'image, l'étape de calcul de valeur de caractéristique et l'étape de classification de phase sont mises en œuvre pour l'ensemble d'une pluralité d'images capturées de la structure métallographique du matériau métallique.

2. Procédé de classification de phase de structure métallographique selon la revendication 1, dans lequel l'étape de calcul de valeur de caractéristique calcule une ou plusieurs valeurs de caractéristique parmi

une valeur de caractéristique d'identité représentant la valeur de luminance de l'image,

une valeur de caractéristique moyenne représentant une valeur moyenne de la valeur de luminance dans une plage prédéterminée de l'image,

une valeur de caractéristique gaussienne représentant la valeur moyenne de la valeur de luminance présentant un poids plus grand à une position plus proche du centre dans la plage prédéterminée de l'image,

une valeur de caractéristique médiane représentant la valeur centrale de la valeur de luminance dans la plage prédéterminée de l'image,

une valeur de caractéristique maximale représentant la valeur maximale de la valeur de luminance dans la plage prédéterminée de l'image,

une valeur de caractéristique minimale représentant la valeur minimale de la valeur de luminance dans la plage prédéterminée de l'image,

une valeur de caractéristique de dérivée représentant une valeur différentielle de la valeur de luminance de l'image, et

une valeur de caractéristique de somme de dérivées obtenue en additionnant la valeur de caractéristique de dérivée.

3. Procédé de classification de phase de structure métallographique selon la revendication 2, dans lequel la plage prédéterminée est une plage dans laquelle, parmi une ou une pluralité de phases désignées à l'avance pour la structure métallographique, une taille inférieure à 1/2 d'une plus petite taille de grain cristallin est incluse et une taille inférieure à 1/2 d'une plus grande taille de grain cristallin est incluse.

4. Procédé de classification de phase de structure métallographique selon l'une quelconque des revendications 1 à 3, dans lequel

le matériau métallique est une tôle d'acier duplex, et

l'étape de classification de phase classe les phases de ferrite et de martensite.

5. Procédé de classification de phase de structure métallographique selon l'une quelconque des revendications 1 à 4, dans lequel le modèle dont l'apprentissage est terminé est un modèle d'apprentissage automatique incluant un arbre de décision, une forêt aléatoire ou un réseau neuronal.

**6.** Dispositif de classification de phase de structure métallographique comprenant :

une unité de prétraitement d'image configurée pour sélectionner une ou un nombre prédéterminé d'images parmi une pluralité d'images et ajuster la valeur de luminance de chaque pixel de chaque image sélectionnée ;
une unité de calcul de valeur de caractéristique configurée pour calculer une ou plusieurs valeurs de caractéristique pour chaque pixel d'une image capturée de la structure métallographique d'un matériau métallique ;
une unité de classification de phase configurée pour classer la phase de la structure métallographique dans l'image en entrant chaque valeur de caractéristique calculée par l'unité de calcul de valeur de caractéristique dans un modèle dont l'apprentissage est terminé ayant été soumis à un apprentissage en utilisant des valeurs de caractéristique auxquelles des libellés d'une phase ou d'une pluralité de phases désignées à l'avance pour la structure métallographique sont attribués en tant qu'entrées et les libellés des phases en tant que sorties, et en acquérant le libellé de la phase d'un pixel correspondant à la valeur de caractéristique d'entrée ; une unité de prétraitement d'image configurée pour ajuster la valeur de luminance de chaque pixel de l'image ;
une unité de valeur d'évaluation d'erreur configurée pour calculer une valeur d'évaluation d'erreur par détermination vrai/faux, pour chaque pixel, de chaque phase de la structure métallographique ; et
une unité d'ajustement de prétraitement configurée pour ajuster un paramètre de prétraitement de l'unité de prétraitement d'image en modifiant le paramètre de prétraitement de telle sorte que la valeur d'évaluation d'erreur calculée par l'unité de valeur d'évaluation d'erreur diminue,
dans lequel le paramètre de prétraitement ajusté par l'unité de réglage de prétraitement est fixe et l'étape de prétraitement d'image, l'étape de calcul de valeur de caractéristique et l'étape de classification de phase sont mises en œuvre pour l'ensemble d'une pluralité d'images capturées de la structure métallographique du matériau métallique.

**7.** Procédé d'apprentissage de phase de structure métallographique destiné à générer le modèle dont l'apprentissage est terminé utilisé dans le procédé de classification de phase de structure métallographique selon l'une quelconque des revendications 1 à 5, le procédé d'apprentissage de phase de structure métallographique comprenant :

une étape de désignation de phase consistant à attribuer l'un des libellés d'une phase ou d'une pluralité de phases désignées à l'avance pour la structure métallographique d'un matériau métallique à un pixel correspondant à la phase dans une image capturée de la structure métallographique ;
une étape de calcul de valeur de caractéristique consistant à calculer une ou plusieurs valeurs de caractéristique pour chacun des pixels auxquels les libellés des phases sont attribués ; et
une étape de génération de modèle consistant à générer un modèle dont l'apprentissage est terminé par un apprentissage utilisant les valeurs de caractéristique auxquelles les libellés des phases sont attribués en tant qu'entrées et les libellés des phases en tant que sorties.

**8.** Dispositif d'apprentissage de phase de structure métallographique destiné à générer le modèle dont l'apprentissage est terminé utilisé dans le dispositif de classification de phase de structure métallographique selon la revendication 6, le dispositif d'apprentissage de phase de structure métallographique comprenant :

une unité de désignation de phase configurée pour attribuer l'un des libellés d'une phase ou d'une pluralité de phases désignées à l'avance pour la structure métallographique d'un matériau métallique à un pixel correspondant à la phase dans une image capturée de la structure métallographique ;
une unité de calcul de valeur de caractéristique configurée pour calculer une ou plusieurs valeurs de caractéristique pour chacun des pixels auxquels les libellés des phases sont attribués ; et
une unité de génération de modèle configurée pour générer un modèle dont l'apprentissage est terminé par un apprentissage utilisant les valeurs de caractéristique auxquelles les libellés des phases sont attribués en tant qu'entrées et les libellés des phases en tant que sorties.

**9.** Procédé de prédiction de propriété de matériau métallique consistant à prédire des propriétés de matériau d'un matériau métallique, le procédé de prédiction de propriété de matériau métallique comprenant, après le procédé de classification de phase de structure métallographique selon l'une quelconque des revendications 1 à 5 :

une étape d'évaluation quantitative consistant à calculer une valeur d'évaluation quantitative d'une structure métallographique en calculant la taille, le rapport de surface ou la forme de chaque phase classifiée ;
une étape de sélection de données consistant à sélectionner des données devant être utilisées pour prédire les propriétés de matériau du matériau métallique parmi la valeur d'évaluation quantitative et les propriétés de matériau du matériau métallique préparées à l'avance ;

une étape de génération de modèle consistant à générer, en utilisant les données sélectionnées, un modèle de prédiction qui prédit les propriétés de matériau du matériau métallique ; et

une étape de prédiction de propriété de matériau métallique consistant à prédire les propriétés de matériau du matériau métallique en utilisant le modèle de prédiction généré.

10. Dispositif de prédiction de propriété de matériau métallique configuré pour prédire des propriétés de matériau d'un matériau métallique, le dispositif de prédiction de propriété de matériau métallique comprenant :

une unité d'entrée configurée pour entrer une image pour laquelle une classification de phase d'une structure métallographique est mise en œuvre par le dispositif de classification de phase de structure métallographique de la revendication 6 ;

une unité d'évaluation quantitative configurée pour calculer une valeur d'évaluation quantitative de la structure métallographique en calculant la taille, le rapport de surface ou la forme de chaque phase classifiée ;

une unité d'enregistrement de données configurée pour enregistrer la valeur d'évaluation quantitative dans une base de données ;

une unité de sélection de données configurée pour sélectionner des données devant être utilisées pour prédire les propriétés de matériau du matériau métallique parmi la valeur d'évaluation quantitative enregistrée dans la base de données et les propriétés de matériau du matériau métallique ;

une unité de génération de modèle configurée pour générer, en utilisant les données sélectionnées, un modèle de prédiction qui prédit les propriétés de matériau du matériau métallique ;

une unité de prédiction de propriété de matériau métallique configurée pour prédire les propriétés de matériau du matériau métallique en utilisant le modèle de prédiction généré ; et

une unité de sortie configurée pour délivrer en sortie les propriétés de matériau prédites du matériau métallique.

# FIG.1

1

## COMPUTATION UNIT 30

INPUT UNIT 10

### IMAGE PREPROCESSING UNIT 31

### PHASE DESIGNATION UNIT 32

### FEATURE VALUE CALCULATION UNIT 33

OUTPUT UNIT 20

### MODEL GENERATION UNIT 34

# FIG.2

2

## COMPUTATION UNIT 60

INPUT UNIT 40

### IMAGE PREPROCESSING UNIT 61

### FEATURE VALUE CALCULATION UNIT 62

OUTPUT UNIT 50

### PHASE CLASSIFICATION UNIT 63

# FIG.3

3

## COMPUTATION UNIT 90

### IMAGE PREPROCESSING UNIT 91

### FEATURE VALUE CALCULATION UNIT 92

### PHASE CLASSIFICATION UNIT 93

### ERROR EVALUATION VALUE CALCULATION UNIT 94

### PREPROCESSING ADJUSTMENT UNIT 95

INPUT UNIT 70

OUTPUT UNIT 80

# FIG.4

START

↓

⌒S1
INPUT IMAGE

↓

⌒S2
PREPROCESS IMAGE

↓

⌒S3
DESIGNATE METALLOGRAPHIC
STRUCTURE PHASE

↓

⌒S4
CALCULATE FEATURE VALUE

↓

⌒S5
GENERATE LEARNING-COMPLETED
MODEL

↓

END

# FIG.5

START

↓

⌒S11
INPUT IMAGE

↓

⌒S12
PREPROCESS IMAGE

↓

⌒S13
CALCULATE FEATURE VALUE

↓

⌒S14
CLASSIFY METALLOGRAPHIC
STRUCTURE PHASE

↓

END

# FIG.6

START

S21
INPUT IMAGE OF PART OF SPECIMEN

S22
PREPROCESS IMAGE

S23
CALCULATE FEATURE VALUE

S24
CLASSIFY METALLOGRAPHIC STRUCTURE PHASE

S25
CALCULATE ERROR EVALUATION VALUE

S26
IS CLASSIFICATION ACCURACY EQUAL TO OR HIGHER THAN THRESHOLD VALUE?

NO

YES

S31
CHANGE PREPROCESSING CONDITIONS

S27
INPUT OTHER IMAGES OF SPECIMEN

S28
PREPROCESS IMAGE

S29
CALCULATE FEATURE VALUE

S30
CLASSIFY METALLOGRAPHIC STRUCTURE PHASE

END

# FIG.7

4

**COMPUTATION UNIT** 120

**INPUT UNIT** 100

**OUTPUT UNIT** 110

**STORAGE UNIT** 130

**QUANTITATIVE EVALUATION UNIT** 121

**DATA RECORDING UNIT** 122

**DATA SELECTION UNIT** 123

**MODEL GENERATION UNIT** 124

**MATERIAL PROPERTY PREDICTION UNIT** 125

# FIG.8

START

S41
INPUT CLASSIFICATION IMAGE

S42
CALCULATE QUANTITATIVE
EVALUATION VALUE

S43
RECORD DATA

S44
SELECT DATA

S45
GENERATE PREDICTION MODEL

S46
PREDICT MATERIAL PROPERTIES

S47
OUTPUT PREDICTION RESULT

END

# FIG.9

(a)

L1

(b)

# FIG.10

(a)

(b)

# FIG.11

| | FERRITE PHASE |
|---|---|
| | MARTENSITE PHASE |

# FIG.12

(a)IDENTITY FEATURE VALUE

(b)Gausian (4) FEATURE VALUE

(c)Mean (4) FEATURE VALUE

# FIG.13

# FIG.14

(a)IDENTITY FEATURE VALUE

220

0

(b)Gausian (16) FEATURE VALUE

220

0

(c)Mean (16) FEATURE VALUE

220

0

# FIG.15

(a)ORIGINAL IMAGE

(b)CLASSIFICATION RESULT OF EXAMPLE
(CLASSIFICATION ACCURACY: GOOD)

☐ FERRITE　■ MARTENSITE

(c)CLASSIFICATION RESULT OF COMPARATIVE
EXAMPLE (CLASSIFICATION ACCURACY: POOR)

☐ FERRITE　■ MARTENSITE

EP 4 099 012 B1

# FIG.16

(a)ORIGINAL IMAGE

(b)AFTER BACKGROUND REMOVAL

(c)CLASSIFICATION RESULT OF EXAMPLE
(CLASSIFICATION ACCURACY: GOOD)

☐ FERRITE   ■ MARTENSITE

(d)CLASSIFICATION RESULT OF COMPARATIVE
EXAMPLE (CLASSIFICATION ACCURACY: POOR)

☐ FERRITE   ■ MARTENSITE

# FIG.17

(a)

(b)

# FIG.18

Legend:
○ TRAINING DATA
□ TEST DATA

Y-axis: NORMALIZED TENSILE STRENGTH (PREDICTED VALUE)

X-axis: NORMALIZED TENSILE STRENGTH (ACTUAL VALUE)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018121752 A

- WO 2017010397 A

**Non-patent literature cited in the description**

- Advanced microstructure classification by data mining methods. **JESSICA GOLA et al.** COMPUTATIONAL MATERIALS SCIENCE. ELSEVIER, 23 March 2018, vol. 148, 324-335